# EUROPEAN PATENT SPECIFICATION

(11) **EP 3 852 767 B1**
(45) Date of publication and mention of the grant of the patent: **17.12.2025**
(21) Application number: 19782483.2
(22) Date of filing: 18.09.2019
(51) Int. Cl.: C07F 9/117, A61P 31/12

(54) **CALCIUM OXALATE CRYSTALLIZATION INHIBITORS FOR RENAL DISORDERS**
CALCIUMOXALAT-KRISTALLISATIONSHEMMER FÜR NIERENERKRANKUNGEN
INHIBITEURS DE CRISTALLISATION D'OXALATE DE CALCIUM POUR TROUBLES RÉNAUX

(30) Priority: 18.09.2018 EP 18195316; 20.03.2019 EP 19164195
(43) Date of publication of application: 28.07.2021
(73) Proprietor: Vifor (International) AG, 9014 St. Gallen (CH)
(72) Inventor: IVARSSON, Mattias Emanuel, 8002 Zürich (CH); LEROUX, Jean-Christophe, 8053 Zürich (CH); KLETZMAYR, Anna, 8049 Zürich (CH)
(74) Representative: Schulz Junghans Patentanwälte PartGmbB
(86) International application number: PCT/EP2019/074986
(87) International publication number: WO 2020/058321

(56) References cited:
- WO-A1-2013/045107
- WO-A1-2017/098047
- BAILON PASCAL ET AL: "PEG-modified biopharmaceuticals", EXPERT OPINION ON DRUG DELI, INFORMA HEALTHCARE, GB, vol. 6, no. 1, 1 January 2009 (2009-01-01), pages 1 - 16, XP008108163, ISSN: 1742-5247, DOI: 10.1517/17425240802650568
- L. ZHANG ET AL.: "Using inositol as a biocompatible ligand for efficient transgene expression", INTERNALTIONAL JOURNAL OF NANOMEDICINE, vol. 10, 1 January 2015 (2015-01-01), pages 2871 - 2884, XP002793147
- RILEY ANDREW M ET AL: "Interactions of inositol 1,4,5-trisphosphate (IP3) receptors with synthetic poly(ethylene glycol)-linked dimers of IP3 suggest close spacing of the IP3-binding sites", JOURNAL OF BIOLOGICAL CHEMISTRY, AMERICAN SOCIETY FOR BIOCHEMISTRY AND MOLECULAR BIOLOGY, US, vol. 277, no. 43, 25 October 2002 (2002-10-25), pages 40290 - 40295, XP002411424, ISSN: 0021-9258, DOI: 10.1074/JBC.M206925200

## Description

### Background of the invention

Kidney stones are mineral deposits in the renal tissue, which form due to supersaturation of the urine with a certain mineral. The prevalence of kidney stones is high with rates up to 14.8%, and a drastic risk of recurrence. Moreover, incidence is on the rise due to changes in lifestyle, diet and the enhanced occurrence of associated risk factors, such as obesity, diabetes and hypertension.

Underlying causes that lead to the formation of kidney stones are diverse. However, one common constituent of the majority of kidney stones is calcium oxalate (CaOx). CaOx crystallization and stone formation in the kidney can physically obstruct the tubules, as well as elicit inflammation and fibrosis of the tissue, together with impairing the organ's function, creating the risk of developing chronic kidney disease and end-stage renal disease.

Another calcium precipitate of clinical importance in the kidney is calcium phosphate. Kidney stones composed predominantly (50% or more) of calcium phosphate constitute up to 10% of all stones and 15%-20% of calcium stones, 80% of which are composed of calcium oxalate. Calcium phosphate is a minor component of up to 30% of calcium oxalate stones as well.

Different diseases, such as hyperoxaluria, Dent disease or Bartter's syndrome, can lead to CaOx kidney stone formation. In the case of primary hyperoxaluria for example, oxalate is produced in excess due to absence or malfunction of liver proteins involved in oxalate metabolism. The high oxalate load in urine causes CaOx crystallization in the renal tubes, kidney stone formation and hence damage of the tissue. Patients suffer from kidney failure and end-stage renal disease at a young age. Due to limited treatment options 80% of patients need combined liver-kidney transplantation at the age of 30.

To date therapeutic options for kidney stone patients are merely palliative and far from disease modifying, thus leaving the patient at risk for stone recurrence and severe complications, such as chronic kidney disease and end stage renal disease. Existing stones can be removed surgically, accompanied by medical management of the pain associated with kidney stones. However, recurrence rate is high and preventive measurements are limited. Suggested measures include high fluid intake, adapted nutrition and thiazides for patients with high urinary calcium levels.

Although CaOx crystallization inhibitors (such as citrate or phosphate, among others) have been proposed to be used to prevent kidney stone formation, limited efficacy and unwanted side effects limit their application in the clinic. Citrate has shown some efficacy as a preventive measurement in recurrent CaOx stone patients, however its mode of action remains unclear. WO2013045107A1 discloses mono-pegylated inositol derivatives and their use in treatment of clostridium infection.

Taken together, a pharmaceutical agent capable of decreasing the propensity for CaOx crystal nucleation, growth and adherence to renal epithelial cells, and ultimately reducing damage of the kidneys independent of the underlying cause, would be significant therapeutic value. A potent inhibitor of CaOx crystallization in urine would be applicable in a variety of disorders associated with kidney stone formation and could decrease the risk of further, severe complications.

Based on the above-mentioned state of the art, the objective of the present invention is to provide pharmaceutical compounds for treating or preventing calcium oxalate and/or calcium phosphate crystallization in conditions related to pathological calcium oxalate crystallization. This objective is attained by the subject-matter of the independent claims of the present specification.

### Description

### Summary of the invention

A first aspect of the invention relates to a compound comprising two or more cyclohexanolpentakisester moieties described by a general formula (I) linked by a common central linker L, 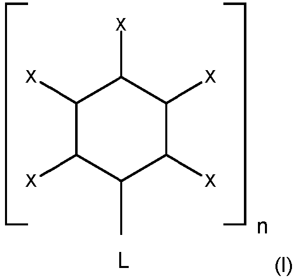 wherein X is OPO₃²⁻ , L is a common central linker to which n individual moieties characterized by the formula in brackets are attached and n is an integer from 2 and 3.

In certain embodiments, the compound is described by the general formula (II) or (III)

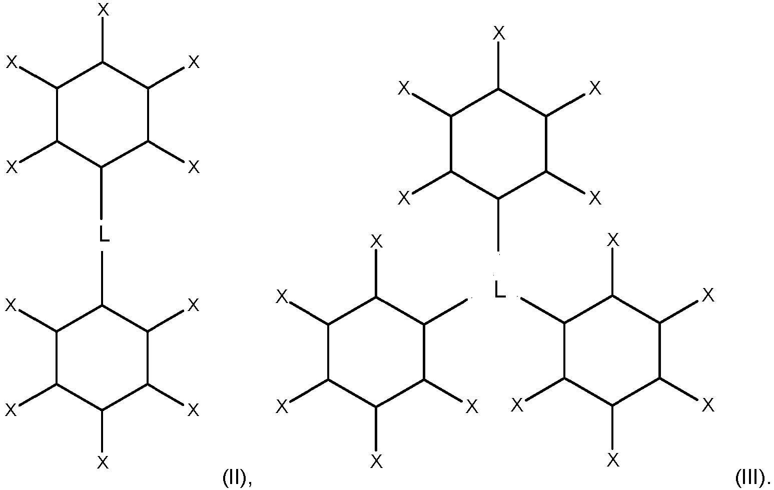

It is understood that any compounds of the invention, where defined herein as a negatively charged compound, will be associated with pharmaceutically acceptable counterions.

A second aspect of the invention relates to a compound as specified herein for use in the treatment of a pathology associated with crystallization, or formation, of calcium precipitates.

Likewise, the invention relates to a compound as specified herein for use in the treatment of a pathology associated with crystallization, or formation, of oxalate precipitates.

### Terms and definitions

The term *C₁-C₄ alkyl* in the context of the present specification signifies a saturated linear or branched hydrocarbon having 1, 2, 3 or 4 carbon atoms. Non-limiting examples for a C₁-C₄ alkyl are methyl, ethyl, propyl, n-butyl, 2-methylpropyl, tert-butyl. In certain embodiments, a C₁-C₄ alkyl is a methyl, ethyl, propyl or butyl moiety.

A C₁-C₆ alkyl in the context of the present specification signifies a saturated linear or branched hydrocarbon having 1, 2, 3, 4, 5 or 6 carbon atoms. Non-limiting examples for a C₁-C₆ alkyl include the examples given for C₁-C₄ alkyl above, and additionally n-pentyl, 2-methylbutyl, 3-methylbutyl, 1,1-dimethylpropyl, 1,2-dimethylpropyl, 1,2-dimethylpropyl. In certain embodiments, a C₅ alkyl is a pentyl or cyclopentyl moiety and a C₆ alkyl is a hexyl or cyclohexyl moiety.

The term *unsubstituted Cₙ alkyl* when used herein in the narrowest sense relates to the moiety -CₙH₂ₙ-if used as a bridge between moieties of the molecule, or -CₙH₂ₙ₊₁ if used in the context of a terminal moiety. It may still contain fewer H atoms if a cyclical structure or one or more (non-aromatic) double bonds are present.

The terms *unsubstituted Cₙ alkyl* and *substituted Cₙ alkyl* include a linear alkyl comprising or being linked to a cyclical structure, for example a cyclopropane, cyclobutane, cyclopentane or cyclohexane moiety, unsubstituted or substituted depending on the annotation or the context of mention, having linear alkyl substitutions. The total number of carbon in the linear chain or cyclical structure adds up to n.

Where used in the context of chemical formulae, the following abbreviations may be used: Me is methyl CH₃, *Et* is ethyl -CH₂CH₃, *Prop* is propyl -(CH₂)₂CH₃ (n-propyl, n-pr) or -CH(CH₃)₂ (iso-propyl, i-pr), *but* is butyl -C₄H₉, -(CH₂)₃CH₃, -CHCH₃CH₂CH₃, -CH₂CH(CH₃)₂ or -C(CH₃)₃.

The term *C₄-C₇ cycloalkyl* in the context of the present specification relates to a saturated hydrocarbon ring having 4, 5, 6 or 7 carbon atoms. Non-limiting examples for a C₄-C₇ cycloalkyl moieties include cyclopropanyl (-C₄H₇), cyclopentanyl (C₅H₉), and cyclohexanyl (C₆H₁₁) moieties. In certain embodiments, a cycloalkyl is substituted by one C₁ to C₄ unsubstituted alkyl moiety. In certain embodiments, a cycloalkyl is substituted by more than one C₁ to C₄ unsubstituted alkyl moieties.

The term *substituted alkyl* in its broadest sense refers to an alkyl as defined above in the broadest sense, which is covalently linked to an atom that is not carbon or hydrogen, particularly to an atom selected from N, O, F, B, Si, P, S, Cl, Br and I, which itself may be -if applicable- linked to one or several other atoms of this group, or to hydrogen, or to an unsaturated or saturated hydrocarbon (alkyl or aryl in their broadest sense). In a narrower sense, *substituted alkyl* refers to an alkyl as defined above in the broadest sense that is substituted in one or several carbon atoms by groups selected from amine NH₂, alkylamine NHR, imide NH, alkylimide NR, amino(carboxyalkyl) NHCOR or NRCOR, hydroxyl OH, oxyalkyl OR, oxy(carboxyalkyl) OCOR, carbonyl O and its ketal or acetal (OR)₂, nitrile CN, isonitrile NC, cyanate CNO, isocyanate NCO, thiocyanate CNS, isothiocyanate NCS, fluoride F, choride CI, bromide Br, iodide I, phosphonate PO₃H₂, PO₃R₂, phosphate OPO₃H₂ and OPO₃R₂, sulfhydryl SH, sulphalkyl SR, sulfoxide SOR, sulfonyl SO₂R, sulphanylamide SO₂NHR, sulphate SO₃H and sulphate ester SO₃R, wherein the R substituent as used in the current paragraph, different from other uses assigned to R in the body of the specification, is itself an unsubstituted or substituted C₁ to C₁₂ alkyl in its broadest sense, and in a narrower sense, R is methyl, ethyl or propyl unless otherwise specified.

The term amino substituted alkyl or hydroxyl substituted alkyl refers to an alkyl according to the above definition that is modified by one or several amine or hydroxyl groups NH₂, NHR, NR₂ or OH, wherein the R substituent as used in the current paragraph, different from other uses assigned to R in the body of the specification, is itself an unsubstituted or substituted C₁ to C₁₂ alkyl in its broadest sense, and in a narrower sense, R is methyl, ethyl or propyl unless otherwise specified. An alkyl having more than one carbon may comprise more than one amine or hydroxyl. Unless otherwise specified, the term "substituted alkyl" refers to alkyl in which each C is only substituted by at most one amine or hydroxyl group, in addition to bonds to the alkyl chain, terminal methyl, or hydrogen.

The term carboxyl substituted alkyl refers to an alkyl according to the above definition that is modified by one or several carboxyl groups COOH, or derivatives thereof, particularly carboxylamides CONH₂, CONHR and CONR₂, or carboxylic esters COOR, with R having the meaning as laid out in the preceding paragraph and different from other meanings assigned to R in the body of this specification.

Non-limiting examples of amino-substituted alkyl include -CH₂NH₂, -CH₂NHMe, -CH₂NHEt, -CH₂CH₂NH₂, -CH₂CH₂NHMe, -CH₂CH₂NHEt, -(CH₂)₃NH₂, -(CH₂)₃NHMe, -(CH₂)₃NHEt, -CH₂CH(NH₂)CH₃, -CH₂CH(NHMe)CH₃, -CH₂CH(NHEt)CH₃, -(CH₂)₃CH₂NH₂, -(CH₂)₃CH₂NHMe, -(CH₂)₃CH₂NHEt, -CH(CH₂NH₂)CH₂CH₃, -CH(CH₂NHMe)CH₂CH₃, -CH(CH₂NHEt)CH₂CH₃, -CH₂CH(CH₂NH₂)CH₃, -CH₂CH(CH₂NHMe)CH₃, -CH₂CH(CH₂NHEt)CH₃, -CH(NH₂)(CH₂)₂NH₂, -CH(NHMe)(CH₂)₂NHMe, -CH(NHEt)(CH₂)₂NHEt, -CH₂CH(NH₂)CH₂NH₂, -CH₂CH(NHMe)CH₂NHMe, -CH₂CH(NHEt)CH₂NHEt, -CH₂CH(NH₂)(CH₂)₂NH₂, -CH₂CH(NHMe)(CH₂)₂NHMe, -CH₂CH(NHEt)(CH₂)₂NHEt, -CH₂CH(CH₂NH₂)₂, -CH₂CH(CH₂NHMe)₂ and -CH₂CH(CH₂NHEt)₂ for terminal moieties and -CH₂CHNH₂-, -CH₂CHNHMe-, -CH₂CHNHEt- for an amino substituted alkyl moiety bridging two other moieties.

Non-limiting examples of hydroxy-substituted alkyl include -CH₂OH, -(CH₂)₂OH, -(CH₂)₃OH, -CH₂CH(OH)CH₃, -(CH₂)₄OH, -CH(CH₂OH)CH₂CH₃, -CH₂CH(CH₂OH)CH₃, -CH(OH)(CH₂)₂OH, -CH₂CH(OH)CH₂OH, -CH₂CH(OH)(CH₂)₂OH and -CH₂CH(CH₂OH)₂ for terminal moieties and -CHOH-, -CH₂CHOH-, -CH₂CH(OH)CH₂-, -(CH₂)₂CHOHCH₂-, -CH(CH₂OH)CH₂CH₂-, -CH₂CH(CH₂OH)CH₂-, - CH(OH)(CH₂CHOH-, -CH₂CH(OH)CH₂OH, -CH₂CH(OH)(CH₂)₂OH and -CH₂CHCH₂OHCHOH- for a hydroxyl substituted alkyl moiety bridging two other moieties.

The term *halogen-substituted alkyl* refers to an alkyl according to the above definition that is modified by one or several halogen atoms selected (independently) from F, CI, Br, I.

The term *fluoro substituted alkyl* refers to an alkyl according to the above definition that is modified by one or several fluoride groups F. Non-limiting examples of fluoro-substituted alkyl include -CH₂F, -CHF₂, -CF₃, -(CH₂)₂F, -(CHF)₂H, -(CHF)₂F, -C₂F₅, -(CH₂)₃F, -(CHF)₃H, -(CHF)₃F, -C₃F₇, -(CH₂)₄F, -(CHF)₄H, - (CHF)₄F and -C₄F₉.

Non-limiting examples of hydroxyl- and fluoro-substituted alkyl include -CHFCH₂OH, -CF₂CH₂OH, - (CHF)₂CH₂OH, -(CF₂)₂CH₂OH, -(CHF)₃CH₂OH, -(CF₂)₃CH₂OH, -(CH₂)₃OH, -CF₂CH(OH)CH₃, -CF₂CH(OH)CF₃, -CF(CH₂OH)CHFCH₃, and -CF(CH₂OH)CHFCF₃.

The term *aryl* in the context of the present specification signifies a cyclic aromatic C₅-C₁₀ hydrocarbon. Examples of aryl include, without being restricted to, phenyl and naphthyl. A heteroaryl is an aryl that comprises one or several nitrogen, oxygen and/or sulphur atoms. Examples for heteroaryl include, without being restricted to, pyrrole, thiophene, furan, imidazole, pyrazole, thiazole, oxazole, pyridine, pyrimidine, thiazin, quinoline, benzofuran and indole.

As used herein, the term *pharmaceutical composition* refers to a compound, or a pharmaceutically acceptable salt thereof, together with at least one pharmaceutically acceptable carrier. In certain embodiments, the pharmaceutical composition is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

A first aspect of the invention relates to a compound comprising two or more cyclohexanolpentakisester moieties described by a general formula (I) linked by a common central linker L, 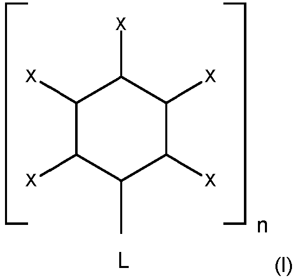 wherein X is OPO₃²⁻, L is a common central linker to which n individual moieties characterized by the formula in brackets are attached and n is an integer from 2 to 3.

The straight lines in formula I and elsewhere herein are meant to indicate that the stereochemistry of the individual ring carbon atoms is undefined. The formula is meant to encompass any diastereomer.

In certain embodiments, n has a value of 2. In certain embodiments n has a value of 3.

The central linker moiety L comprises or consists of a linear or branched poly(ethylene glycol) or polyglycerol.

The linking moiety L has a molecular weight <1000 g/mol, particularly <700 g/mol, more particularly <500 g/mol, or even <400 g/mol.

In certain embodiments, the compound of the invention consists of the central linker L as defined above, and the two or more moieties described by the general formula (I).

All of the X of all moieties (I) comprised in the compound according to the invention are OPO₃²⁻.

In certain embodiments, the linking moiety L comprises or consists of a linear or branched poly(ethylene glycol) or polyglycerol having a molar mass <1000 g/mol, particularly <700 g/mol, more particularly <500 g/mol, or even <400 g/mol.

In certain embodiments, the linking moiety comprises a central core A, to which polyglycerol or poly(ethylene glycol) chains separating the cyclohexanolester moieties of formula (I) are attached.

In certain embodiments, the central core A, if present, can be selected from a substituted or unsubstituted C₁ alkyl, a substituted or unsubstituted C₄ to C₇ cycloalkyl, or a substituted or unsubstituted six-membered aryl.

The linking moiety is generally a simple, metabolically compatible linker designed to facilitate assembly of two or more cyclohexanolpentakisester moieties in the compound at relatively low cost and synthetic ease. In certain embodiments, the linker moiety may be a hydrocarbon substituted by substituents other than hydrogen, for example by one or more substituents selected from oxygen, nitrogen, fluorine or other substituents known in the art of medicinal chemistry to modulate the absorption, distribution, metabolic processing, excretion or toxicity qualities of a compound.

For embodiments wherein the linking moiety L is or comprises a substituted C₁ alkyl, a substituted C₄ to C₇ cycloalkyl, or a substituted six-membered aryl, the substitution may be one or several functions comprising oxygen (by non-limiting example: -OH or =O), one or several functions comprising nitrogen (by non-limiting example: NH₂, NHR^{N}, NR^{N}₂, with R^{N} selected from unsubstituted C₁ to C₄ alkyl), one or several fluorine substituents, or any combination of oxygen, nitrogen or fluorine substituents.

In certain embodiments, any one of the cyclohexanolpentakisester moieties described by the general formula (I) is independently selected from (Ia) and (Ib) 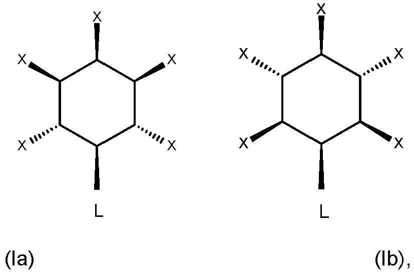 wherein each X (independently) has the meaning outlined above, and wherein L is a linking moiety.

In certain embodiments, the compound is described by the general formula (II), 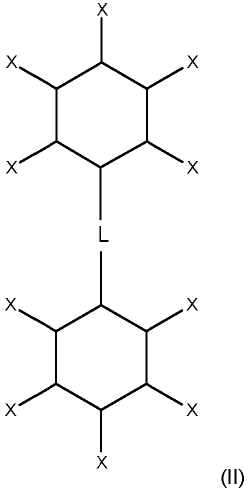 wherein each X independently from any other X is OPO₃²⁻, and wherein L is a common central linker (also referred to as a "linking moiety" herein) as specified above.

In certain embodiments, the compound is described by the general formula (IIi), (IIii), or (IIiii): 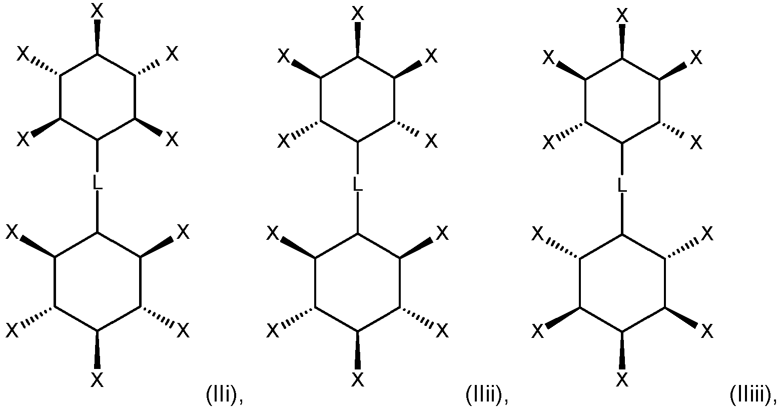 wherein each X independently from any other X is OPO₃²⁻, and wherein L is a common central linker (also referred to as a "linking moiety" herein) as specified above.

In certain embodiments, the compound is described by the general formula (II), (IIi), (IIii), or (IIiii), wherein the linking moiety L is selected from a poly(ethylene glycol) described by a formula -(O-CH₂-CH₂)ₘ-O-, and m has a value from a value between 1 and 12. In particular embodiments, m is selected from 2, 3, or 4.

In certain embodiments the linking moiety L is selected from a polyglycerol described by a formula-(O-CH₂-CH(OH)-CH₂)ₘ-O-, and m has a value from a value between 1 and 12. In particular embodiments, m is selected from 2, 3, or 4.

In certain embodiments, all moieties described by the general formula (I) are either (la) or (Ib).

In certain embodiments, the compound is described by formula (II), (IIi), (IIii), or (IIiii), each X is OPO₃²⁻ and the linking moiety L is a linear poly(ethylene glycol) or polyglycerol.

In certain embodiments, the compound described by formula (II), (IIi), (IIii), or (IIiii) has a molecular weight between 1000 g/mol to 1500 g/mol.

In certain embodiments, the compound is described by formula (II), (IIi), (IIii), or (IIiii), each X is OPO₃²⁻ , the linking moiety L is a linear poly(ethylene glycol) or polyglycerol and the compound has a molecular weight between 1000 g/mol to 1500 g/mol.

In certain embodiments, the compound is described by the general formula (IIiv), (IIv), or (IIvi): 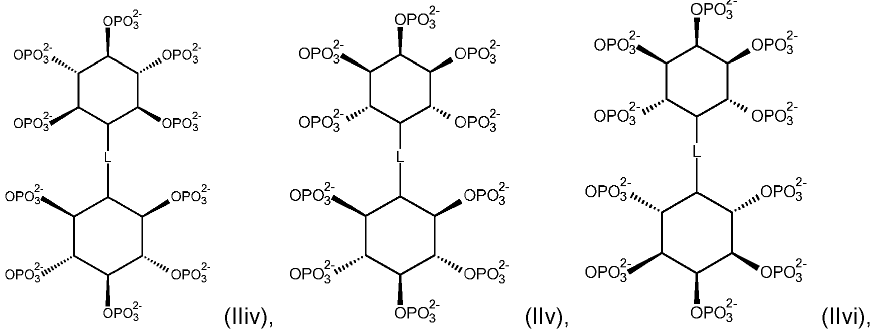 wherein L is a common central linker (also referred to as a "linking moiety" herein) as specified above. In certain embodiments, the compound is described by the general formula (llvii), (llviii), or (IIix):

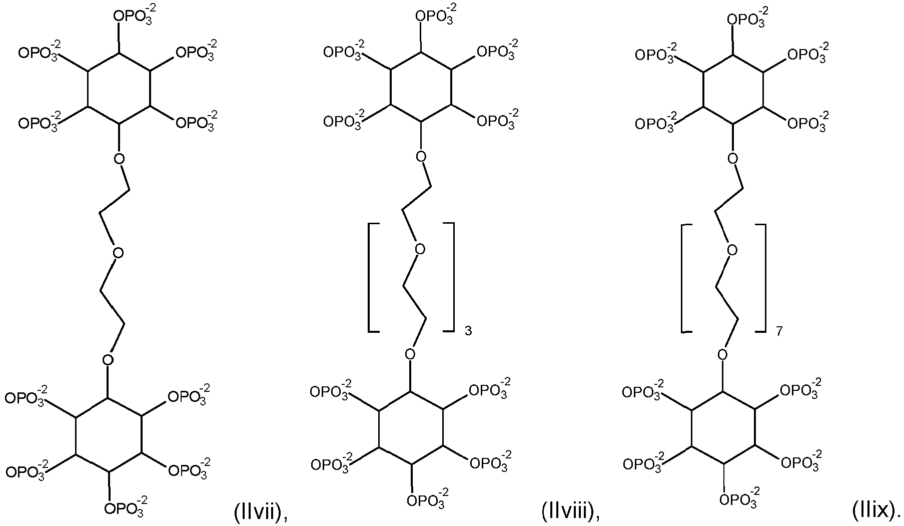

In certain embodiments, the compound is described by formula (IIa), (IIb), (IIc) or (IId):

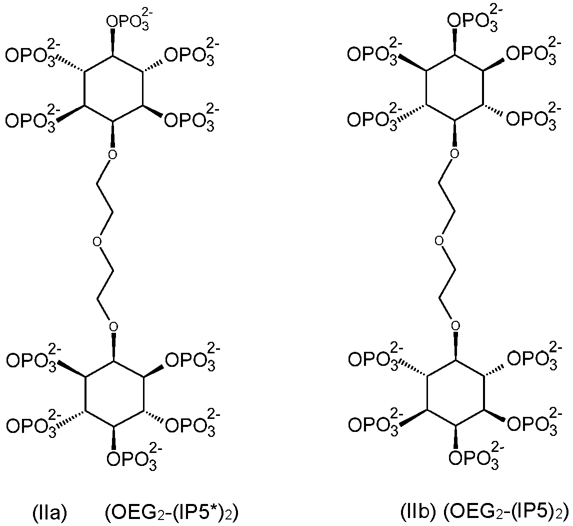

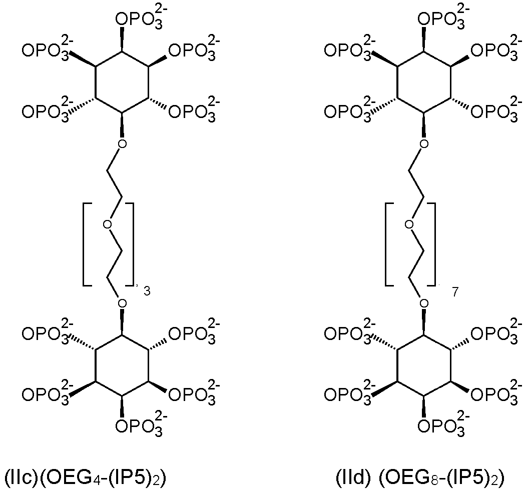

In certain embodiments, the compound is described by formula (lle) or (IIf):

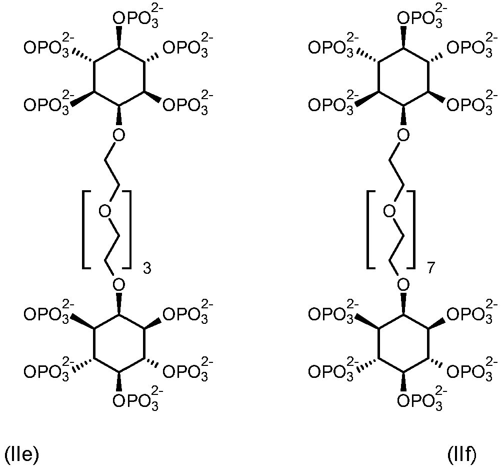

In certain embodiments, the compounds (IIa) (OEG₂-(IP5*)₂) and (IIb) (OEG₂-(IP5)₂) have a molecular mass of 1204.04 g/mol. In one embodiment, the compounds (IIc) (PO1-1) and (lle) have a molecular mass of 1298.15 g/mol. In one embodiment, the compounds (IId) (OEG₈-(IP5)₂) and (IIf) have a molecular mass of 1474.36 g/mol.

In certain embodiments, the compound is described by formula (Ilk), (IIm), or (IIn):

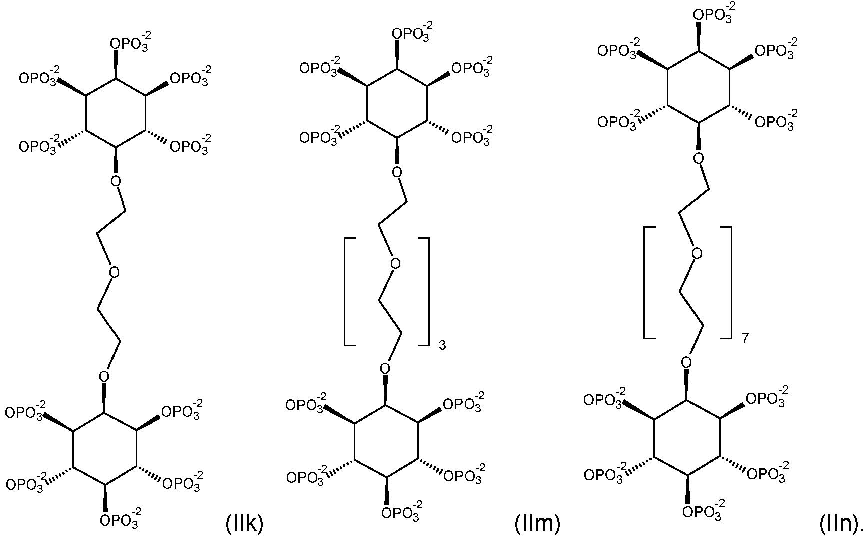

In certain embodiments, the compound of the invention is described by formula (IIg), (IIh), or (IIj) 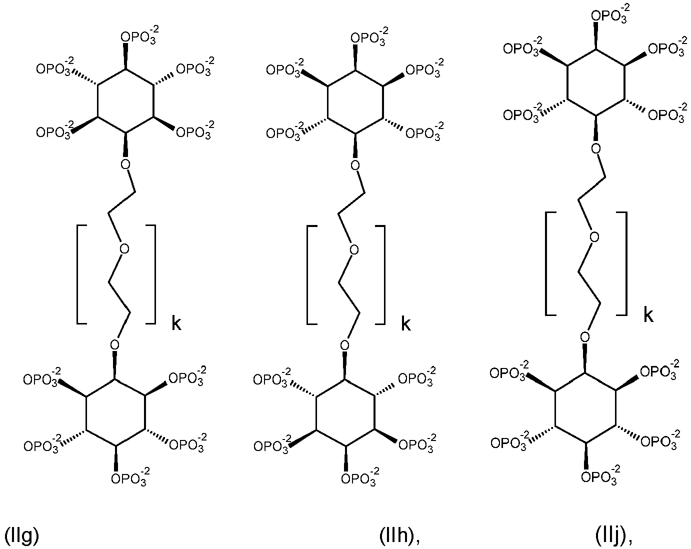 wherein k is an integer selected from 1, 2, 3, 4, 5, 6, 7, 8, 9, 10, 11 and 12.

In certain embodiments, the compound of the invention is described by a general formula (III), 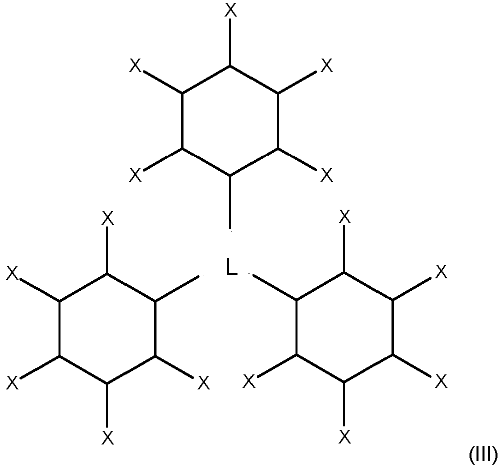 wherein each X independently from any other X is OPO₃²⁻, and wherein L is a common central linker (also referred to as a "linking moiety" herein) as specified above.

In certain embodiments of the compound described by (III), the linking moiety L is described by a general formula (Illa) 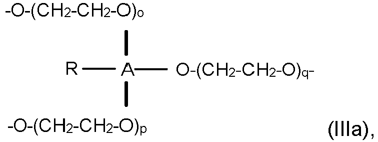 wherein o, p and q independently of each other have a value between 1 and 12, and the sum of o, p and q is ≤ 30.

In certain embodiments, A is selected from a carbon atom (C), a hydroxy-, amino-, halogen- or carboxy-substituted or an unsubstituted C₄ to C₇ cycloalkyl moiety or a hydroxy-, amino-, halogen- or carboxy-substituted or an unsubstituted aryl. In certain embodiments, A is a carbon atom.

In certain embodiments, R is selected from H and C₁-C₃ unsubstituted or N-, O and/or halogen substituted alkyl. In certain embodiments, is H or unsubstituted alkyl.

In certain particular embodiments of (III), L is described by (Illa) and o, p and q independently of each other have a value between 2 and 4.

In certain particular embodiments of (III), L is described by (IIIa) and the sum of o, p and q is ≤ 15.

In certain particular embodiments of (III), L is described by (IIIa) and o, p and q independently of each other have a value between 2 and 4 and the sum of o, p and q is ≤ 15.

In certain embodiments, R is selected from H and C₁-C₃ unsubstituted or N-, O and/or halogen substituted alkyl. In certain particular embodiments, R is H or unsubstituted alkyl.

In certain embodiments of the compound (III), the linking moiety L is described by a general formula (IIIb) 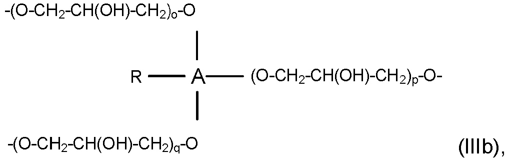 wherein o, p and q independently of each other have a value between 1 and 12, and the sum of o, p and q is ≤ 30.

In certain embodiments, A is selected from a carbon atom (C), a hydroxy-, amino-, halogen- or carboxy-substituted or an unsubstituted C₄ to C₇ cycloalkyl moiety or a hydroxy-, amino-, halogen- or carboxy-substituted or an unsubstituted aryl. In certain embodiments, A is a carbon atom.

In certain embodiments, R is selected from H and C₁-C₃ unsubstituted or N-, O and/or halogen substituted alkyl. In certain embodiments, is H or unsubstituted alkyl.

In certain particular embodiments of (III), L is described by (IIIb) and o, p and q independently of each other have a value between 2 and 4.

In certain particular embodiments of (III), L is described by (IIIb) and the sum of o, p and q is ≤ 15.

In certain particular embodiments of (III), L is described by (IIIb) and o, p and q independently of each other have a value between 2 and 4 and the sum of o, p and q is ≤ 15.

In certain embodiments, of (III), L is described by (IIIa) or (IIIb) and A is a carbon atom. In certain embodiments, of (III), L is described by (IIIa) or (IIIb) and A is selected from a substituted or unsubstituted C₄ to C₇ cycloalkyl. In certain embodiments, of (III), L is described by (IIIa) or (IIIb) and A is selected from a substituted or unsubstituted six-membered aryl.

In certain embodiments of the compound (III), the linking moiety L is described by a general formula (IIIc) 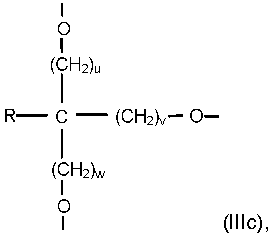 wherein A of formula (Illb) is a carbon atom (C) and u, v and w independently of each other have a value between 1 and 12, particularly u, v and w independently of each other have a value between 2 and 4; the sum of u, v and w is ≤ 30, particularly the sum u, v and w is ≤ 12.

In certain embodiments, R is selected from H and C₁-C₃ unsubstituted or N-, O and/or halogen substituted alkyl, particularly wherein R is H or unsubstituted alkyl.

In certain embodiments, the compound described by general formula (III) has a molecular weight between 1500 to 2500 g/mol.

In certain embodiments, the compound is described by formula (Illd) or (Ille):

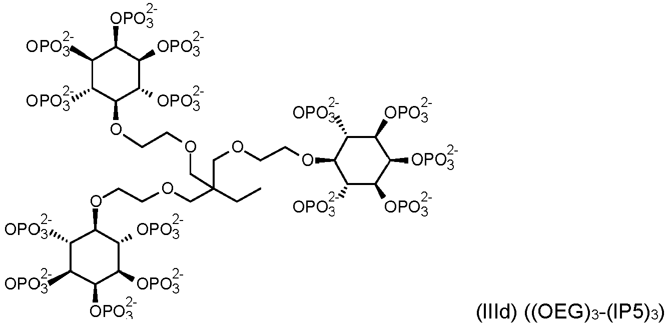

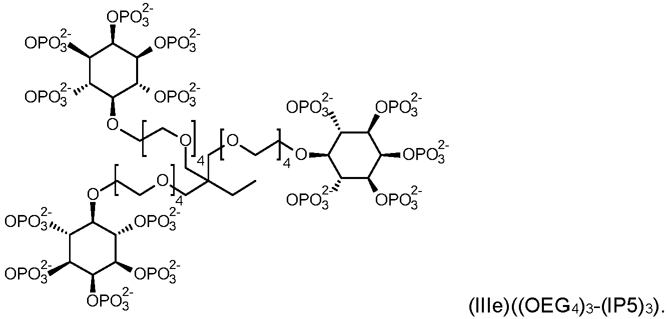

In one embodiment, the compound (Illd) ((OEG)₃-(IP5)₃) has a molecular weight of 1922.21 g/mol. In one embodiment, the compound (Ille) ((OEG₄)₃-(IP5)₃) has a molecular weight of 2318.69 g/mol.

In certain embodiments, the compound of the invention is provided as a sodium salt. In certain embodiments, the compound of the invention is provided as a potassium salt. In certain embodiments, the compound of the invention is provided as a magnesium salt.

The invention further encompasses intermediary products useful in the synthesis of the compounds of the invention, specifically a compound described by general formula Va, Vb or Vc 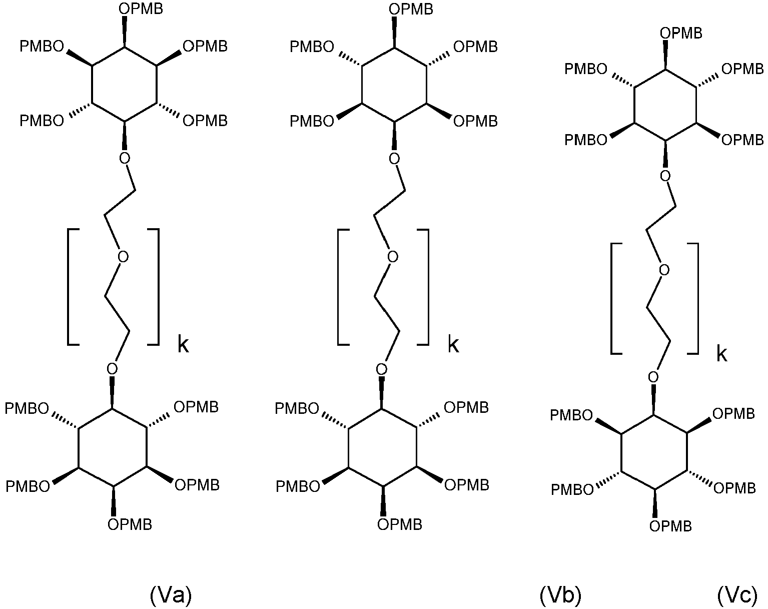 wherein OPMB signifies an O-(bis-paramethoxybenzyl)phosphate moiety and wherein k is an integer selected from 1, 2, 3, 4, 5, 6, 7, 8, 9, 10, 11 and 12.

Another compound useful in the synthesis of the invention is described by the formulae Via, VIb or VIc: 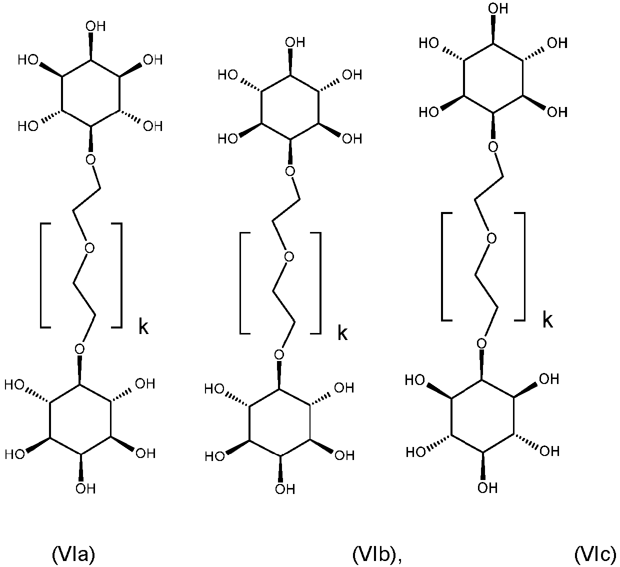 wherein k is an integer selected from 1, 2, 3, 4, 5, 6, 7, 8, 9, 10, 11 and 12.

In one embodiment, the compound (IVd) ((OEG)₄-(IP5)₄) has a molecular weight of 2520.20 g/mol. In one embodiment, the compound (IVe) ((OEG₄)₄-(IP5)₄) has a molecular weight of 3048.84 g/mol.

Another aspect of the invention relates to the use of a compound of the invention as a medicament.

A second aspect of the invention relates to the compound of the invention for use in therapy or prevention of a condition related to pathological crystallization, particularly pathological crystallization of calcium salts and more particularly of calcium oxalate and/or calcium phosphate salts.

Conditions related to pathological calcium crystallization due to pathological calcium oxalate or phosphate crystallization in a patient for which the compounds of the present invention are particularly useful include nephrocalcinosis, nephrolithiasis, calcinosis cutis, kidney stones, cardiovascular mortality (particularly in chronic kidney disease patients), progression of chronic kidney disease and failure of renal transplant grafts. Pathological crystallization has been shown to be associated with morbidity of chronic kidney disease patients, particularly wherein the condition is primary and secondary hyperoxaluria, Dent disease, Bartter's syndrome, distal renal tubule acidosis, neurogenic bladder, autosomal dominant polycystic kidney, calcium oxalate kidney stone formation, struvite stones and renal calcification. Hence the compounds of the present invention are indicated for chronic kidney disease patients in general.

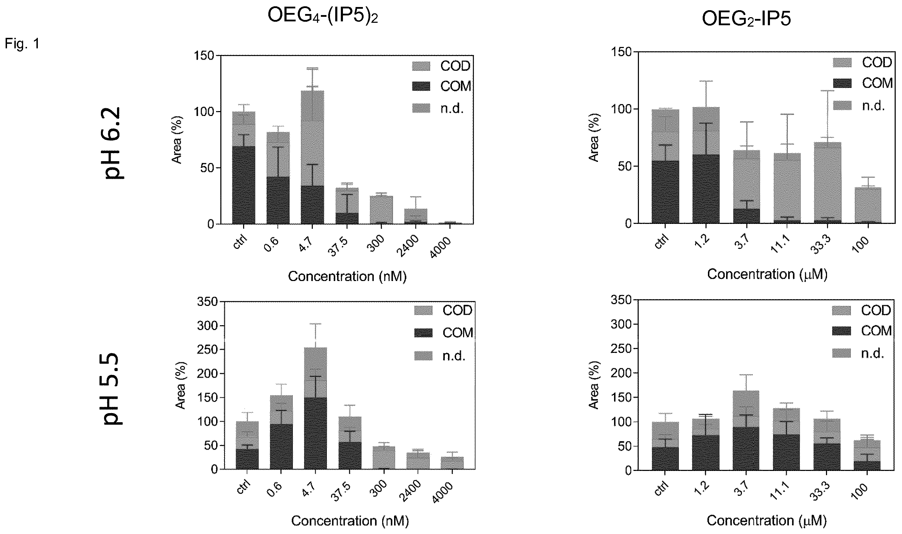

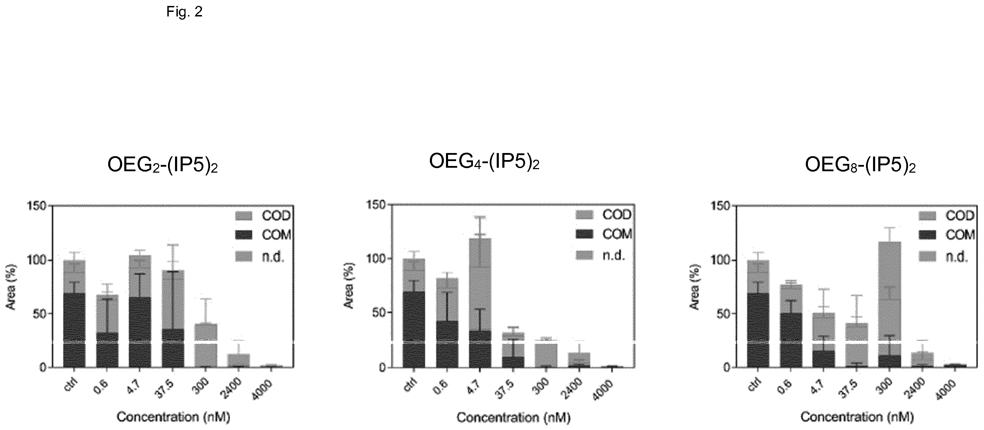

One embodiment of the invention that shows particular high activity is the use of novel divalent IP5 compounds (OEG₄-(IP5)₂, OEG₈-(IP5)₂, OEG₂-(IP5)₂) with similar CaOx inhibitory efficacy (Fig. 2) for the indications listed above. The inventors previously developed a series of small molecules based on inositol hexaphosphate (IP6) analogues (PCT/EP2012/004088, PCT/EP2016/080657) that can decrease the nucleation and growth of CaOx crystals in human urine, and have improved pharmacokinetic (PK) properties. Further, those compounds have been shown to prevent adhesion of CaOx crystals to renal epithelial cells and displayed cyto-protective effects upon CaOx treatment in vitro. While these initial IP6 analogues failed to completely inhibit CaOx crystallization in an in vivo achievable concentration range, the inventors surprisingly found that the potency to inhibit CaOx crystallization was highly dependent on the combination of IP5 moieties. Thus, the inventors developed novel multivalent IP5 compounds by linking two inositol pentaphosphate (IP5) molecules using oligo(ethylene glycol) linkers (OEG), thereby increasing the total negative charge of the molecules. Compared to the previously developed, most potent compound IP5-mono-PEG2 (OEG₂-IP5), the bis-IP5 linked by tris(ethylene glycol) compound OEG₄-(IP5)₂ showed a 2500-fold increase in potency to inhibit CaOx crystallization to below 50% of the control (Fig. 1). This conclusion is drawn from the results of an in vitro assay that measures the propensity for CaOx crystallization of human urine.

The compound may be formulated for intravenous, intraperitoneal, intramuscular, intra-arterial, topical, intravesical or, particularly, subcutaneous administration. Optionally, a pharmaceutically acceptable carrier and/or excipient may be present.

According to another aspect of the invention, a method of treatment or prevention of any of the conditions related to pathological calcium crystallization listed above is provided, comprising the administration of the compound as specified by any of the above formulae to a subject in need thereof.

The skilled person is aware that any compound specifically mentioned herein will be present as a pharmaceutically acceptable salt of the compound. Pharmaceutically acceptable salts comprise the ionized compound and an oppositely charged counterion. Non-limiting examples of pharmaceutically acceptable cationic salt forms include aluminium, benzathine, ethylene diamine, lysine, magnesium, meglumine, potassium, procaine, sodium, tromethamine and zinc.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention.

### Brief description of the figures

- Fig. 1: shows the inhibition of CaOx crystallization by IIc (OEG₄-(IP5)₂) and the comparison IP5-mono-PEG₂ (OEG₂-IP5) at pH 6.2, and at pH 5.5. CaOx crystallization was induced by adding 1 mM sodium oxalate to humane urine and characterized by light microscopy, followed by an automated image analysis to determine crystal number, hydrate forms (COM - CaOx monohydrate, COD - CaOx dihydrate, n.d.- not defined), and crystal area. Surface area of the different hydrate forms normalized to the total surface area of the control is plotted. IIc/ OEG₄-(IP5)₂ led to complete inhibition of COM crystals only at 300 nM, followed by complete CaOx (COM and COD) inhibition at 4 µM. In comparison, for the previous lead compound IP5-mono-PEG₂ COM only inhibition was observed at 11 µM, while complete CaOx (COM and COD) inhibition was not observed in the tested range up to 100 µM.
- Fig. 2: shows the inhibition of CaOx crystallization by compounds of the present invention: IIb (OEG₂-(IP5)₂), IIc (OEG₄-(IP5)₂) and IId (OEG₈-(IP5)₂) at pH 6.2.

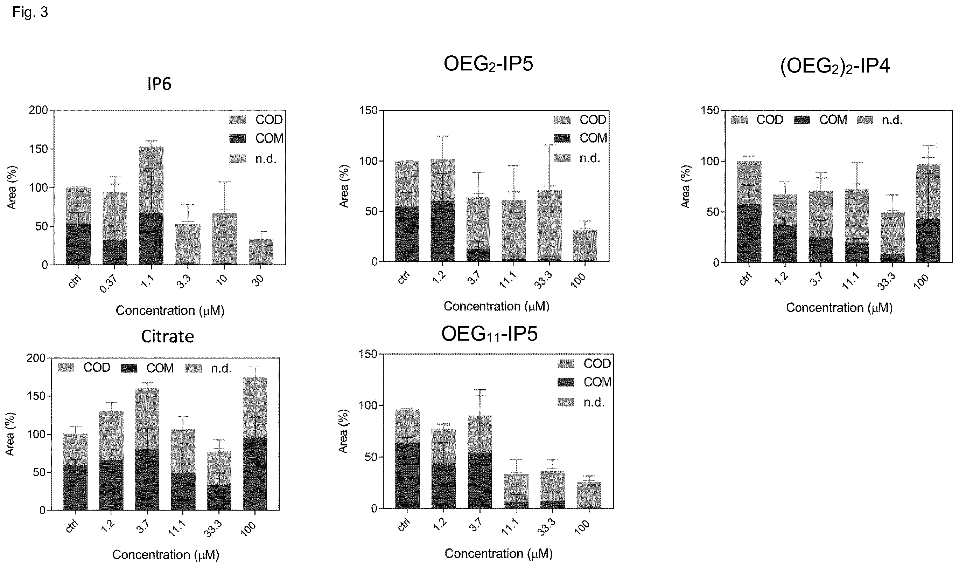

- Fig. 3: shows the inhibition of CaOx crystallization by IP6 analogues previously described in PCT/EP2016/080657 at pH 6.2. CaOx crystallization was induced by adding 1mM sodium oxalate to humane urine and characterized by light microscopy, followed by an automated image analysis to determine crystal number, hydrate forms (COM- CaOx monohydrate, COD - CaOx dihydrate, n.d.- not defined), and crystal area. Surface area of the different hydrate forms normalized to the total surface area of the control is plotted.

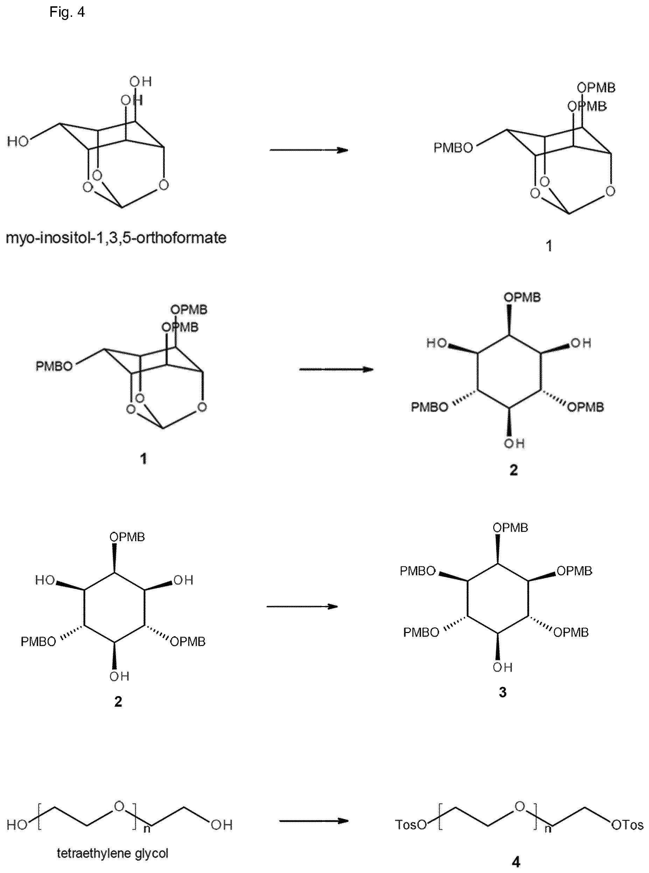

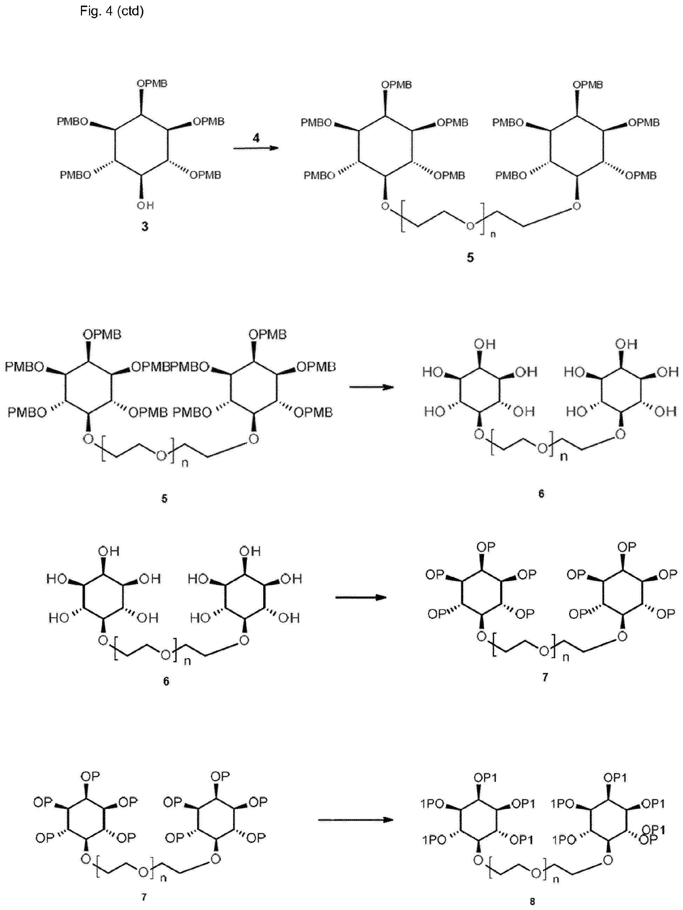

- Fig. 4: shows the synthesis of compounds of the invention as described in example 1.

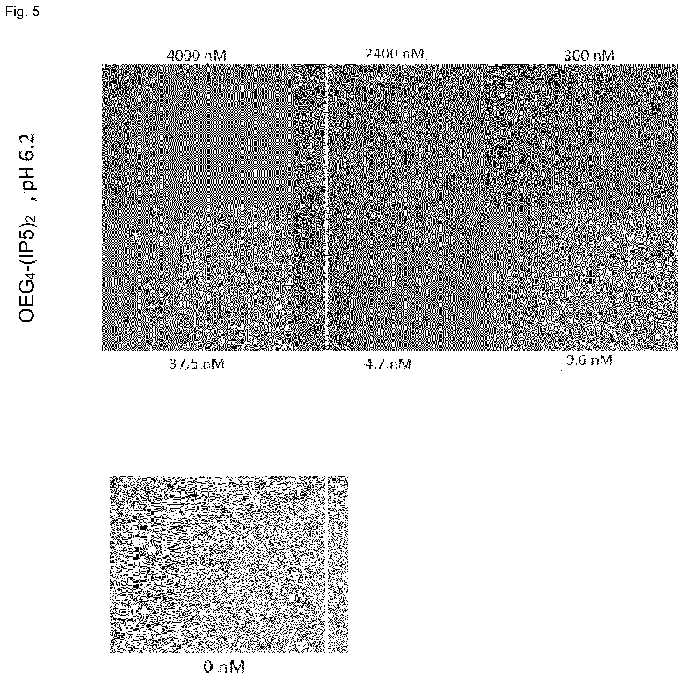

- Fig. 5: shows images of CaOx crystal formation inhibition by a compound of the present invention OEG₄-(IP5)₂ (scale bar: 50 µm).

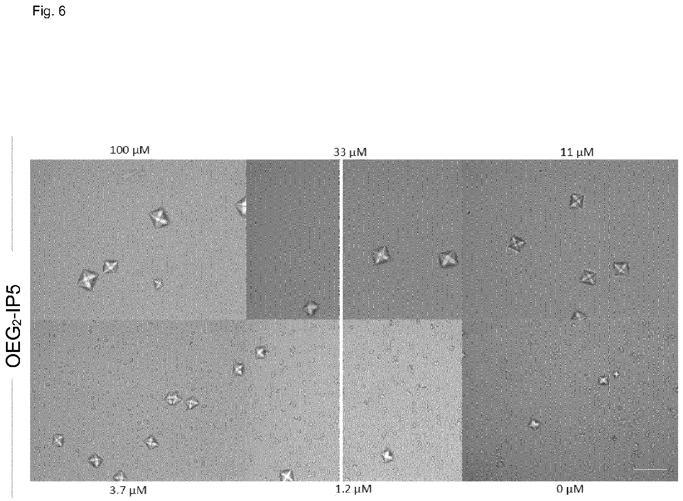

- Fig. 6: shows images of CaOx crystal formation inhibition by an IP6 analogue compound OEG₂-IP5 previously described in PCT/EP2016/080657 (scale bar: 50 µm).

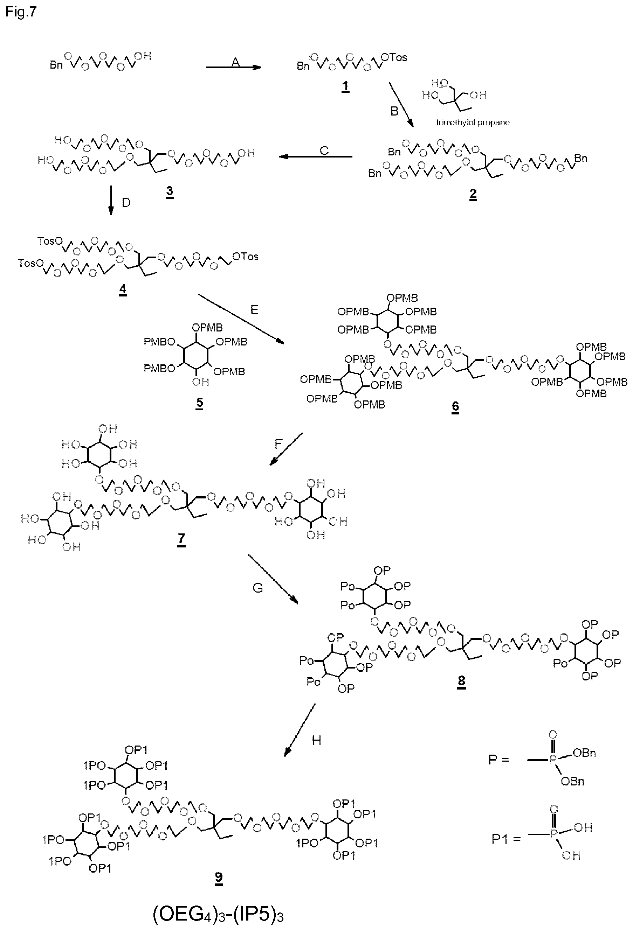

- Fig. 7: shows the synthesis of compound (OEG₄)₃-(IP5)₃.

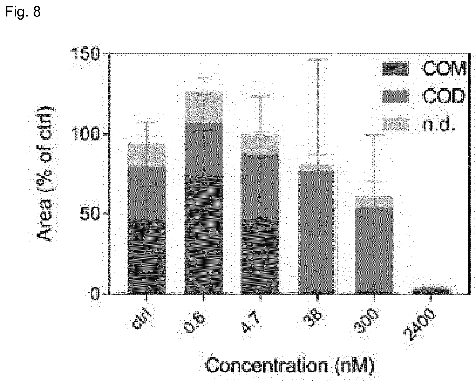

- Fig. 8: shows changes in the CaOx crystallization pattern induced by IP6 analogues. Effects of (OEG₄)₃-(IP5)₃ on CaOx bulk crystallization in human urine spiked with 1 mM NaOx were assessed by light microscopy at t = 7 h. COM - CaOx monohydrate, COD - CaOx dihydrate, n.d. - not defined; N = 3, mean total area / field of view + SD for the respective crystal type normalized to the control (without inhibitor).

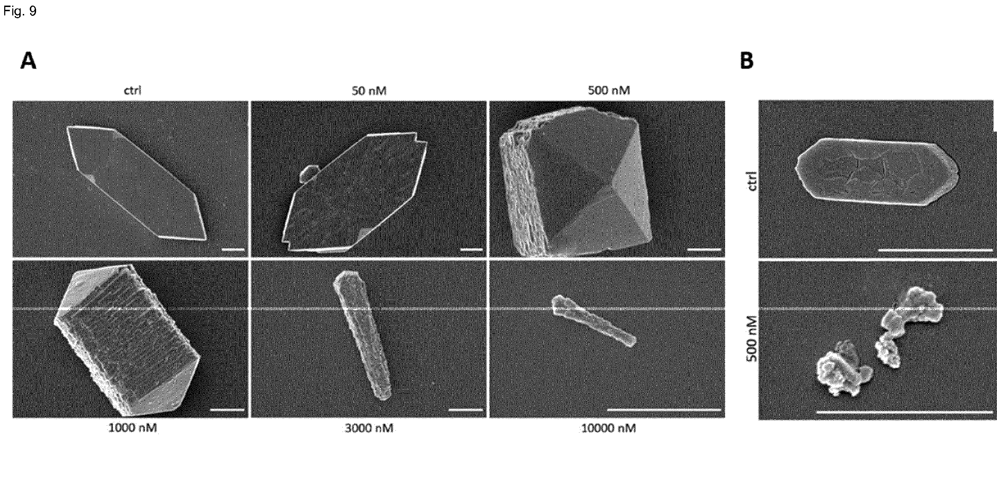

- Fig. 9: shows that OEG₄-(IP5)₂ stabilizes early precursor particles and leads to delayed COD growth. (A) Dose-dependent inhibition of CaOx crystallization by OEG₄-(IP5)₂ in Bis-Tris buffer (pH 6.2) was characterized by SEM at t = 7 h. Representative SEM images are shown (scale bar: 1 µm). (B) Crystallization of CaOx with 500 nM OEG₄-(IP5)₂ and without inhibitor was sampled at t = 10 min and imaged by SEM (scale bar: 1 µm). Crystallinity was characterized by TEM with SAED (inlets).

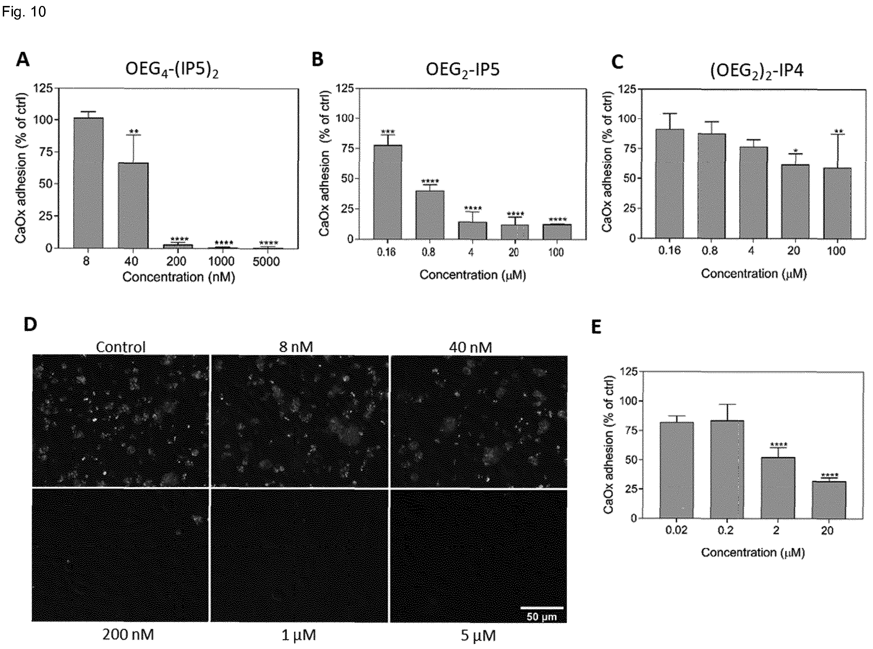

- Fig. 10: shows inhibition of CaOx adhesion to renal epithelial cells by IP6 analogues. Anti-adhesive effects of (A) OEG₄-(IP5)₂, (B) OEG₂-IP5 and (C) (OEG₂)₂-IP4 on CaOx attachment to RPTEC/TERT1 cells *in vitro* were assessed by differential interference contrast microscopy followed by quantification of the crystal occupied area. Cells at confluence were treated with 150 µg/cm² CaOx premixed with compound for 30 minutes, before washing, fixation and imaging. (D) Example images of CaOx premixed with/without OEG₄-(IP5)₂ adhering to the confluent cell layer (Leica CTR6000, 63x oil objective). (E) To assess the capacity of OEG₄-(IP5)₂ to remove already bound CaOx of cell layers an adapted version of the assay described above was performed. RPTEC/TERT1 cells were incubated with CaOx for 30 minutes, unbound CaOx was removed by washing with PBS and cells were further incubated with OEG₄-(IP5)₂ or medium control for 2 h. (N = 3, mean + SD normalized to the CaOx ctrl, one-way ANOVA with Dunnett's multiple comparison, **** p < 0.0001, *** p < 0.001, ** p < 0.01, * p < 0.05, compared to CaOx control.)

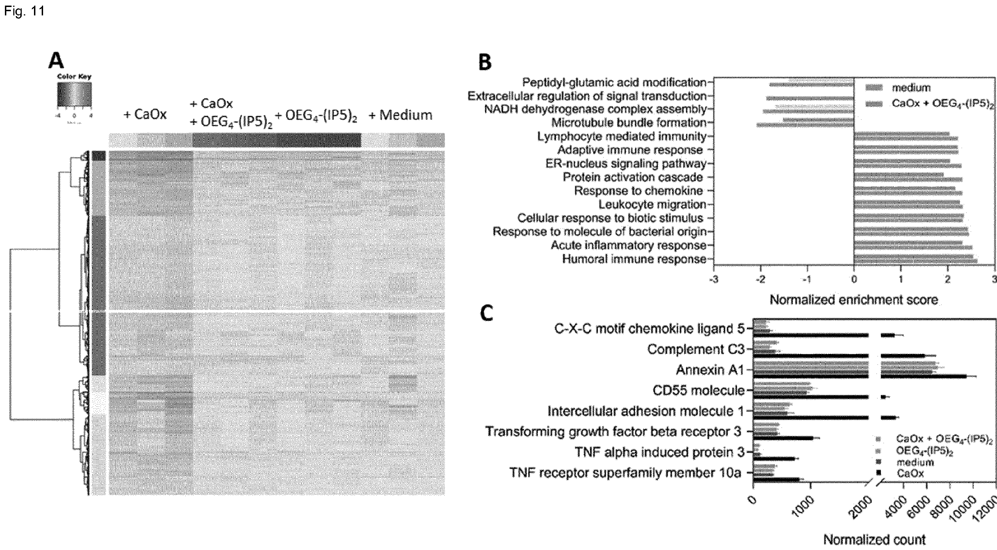

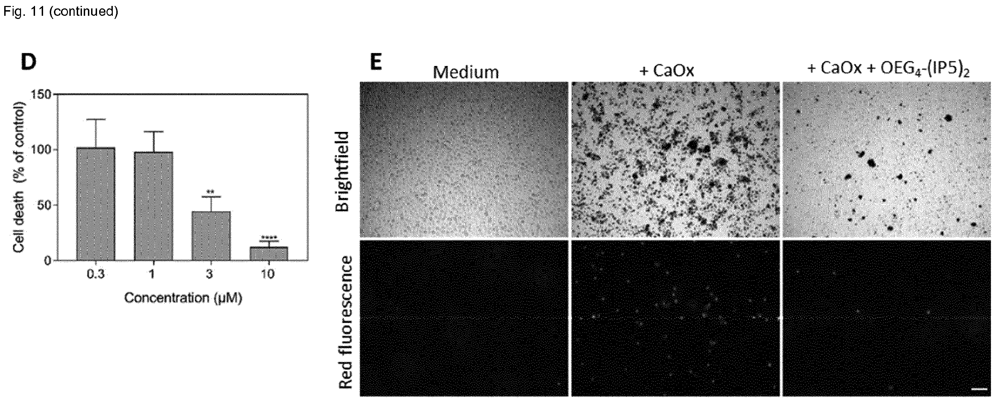

- Fig. 11: shows that CaOx induced cellular injury of renal epithelial cells is prevented by OEG₄-(IP5)₂ *in vitro.* (A) Heatmap and hierarchical clustering of relative gene expression levels determined by RNA sequencing (differentially expressed genes between CaOx versus CaOx - OEG₄-(IP5)₂ with p<0.01, log₂ratio = 0.5, number of significant features 2000). (B) Gene set enrichment analysis of gene expression levels in the CaOx relative to the medium group and corresponding values in the CaOx - OEG₄-(IP5)₂ group. Top gene ontology terms and corresponding normalized enrichment score for differentially up- or down-regulated genes in the CaOx group relative to the medium control group are plotted (top 10 upregulated gene ontology terms, all FDR ≤ 0.05 and downregulated gene ontology terms with FDR ≤ 0.05 are shown). Corresponding values for differentially expressed genes in the CaOx - OEG₄-(IP5)₂ group are given (FDR ≤ 0.05 is indicated by dark blue, FDR > 0.05 by light blue).(C) Gene expression levels of genes involved in inflammatory and immune response, cellular signalling and ECM production in the different treatment groups. (D) Dose-dependent decrease in the number of dead cells by pre-incubation of CaOx with OEG₄-(IP5)₂ before treatment of RPTEC cells in comparison to CaOx only treated cells was assessed by a viability stain (mean + SD normalized to CaOx ctrl, one-way ANOVA with Dunnett's multiple comparison, **** p < 0.0001, ** p <0.01). (E) Example brightfield images showing CaOx deposition (black spots) and red fluorescence images indicating cell death of cells receiving no treatment, CaOx only or CaOx + 10 µM OEG₄-(IP5)₂ treatment (Leica CTR6000, 10x objective, scale bar: 100 µm). (N = 3 for all experiments.)

- Table 1: shows data from a screening of calcification inhibitors for activity in inhibiting growth of CaOx crystals. IP6 analogues previously described in PCT/EP2016/080657 and compounds OEG₂-(IP5)₂, OEG₄-(IP5)₂, OEG₈-(IP5)₂ and (OEG₄)₃-(IP5)₃ of the present invention are listed.

### Examples

### Example 1: Synthesis of IIc (OEG₄-(IP5)₂)

*Step 1:*
   Reagents: DMF, sodium hydride, p-methoxybenzyl chloride.
   Myo-inositol-1,3,5-orthoformate is dissolved in dry DMF under nitrogen atmosphere, cooled to 0-5 °C and added portion-wise with sodium hydride (2.2 eq.). After stirring 1 h at room temperature, p-methoxybenzyl chloride (2.3 eq.) is added dropwise. The mixture is stirred at room temperature for 12 h, then it is added with water and extracted with ethyl acetate. Solvent removed under vacuum and residue crystallized with diethyl ether. Yield 70%.
*Step 2:*
   Reagents: MeOH; HCl 2N.
   The product of step 1 (1) is dissolved in 10 vol of methanol, added with 1 vol of HCl 2 N and heated at 50 °C for 3 hs. After cooling, the mixture is neutralized with ammonium hydroxide 30% and left overnight at room temperature. The solid formed, is filtered, washed with water and dried. After crystallization with diethyl ether compound 2 is obtained as whitish solid. Yield 80%.
*Step 3:*
   Reagents: DMF, sodium hydride, p-methoxybenzyl chloride.
   The product of step 2 (2) is dissolved in dry DMF under nitrogen atmosphere, cooled at 0-5 °C and added with sodium hydride (2 eq.) portion-wise. After 1 h at room temperature, the mixture is cooled at 0-5 °C and added with p-methoxybenzyl chloride (2 eq.). After 12 h at room temperature, the mixture is added with water and extracted with ethyl acetate. The residue is purified by column chromatography eluting with hexanes/ethyl acetate 6/4, then crystallized with diethyl ether.Yield 35%.
*Step 4:*
   Reagents: dichloromethane, Tosyl chloride, triethylamine.
   Tetraethylene glycol is dissolved in dry dichloromethane, added with triethylamine (3 eq.) and then with p-toluenesulfonyl chloride (2.5 eq.). After stirring for 12 h at room temperature, the solvent is removed under vacuum and the residue purified by column chromatography, eluting with ethyl acetate/hexanes 7/3. Yield 80%.
*Step 5:*
   Reagents: DMF, sodium hydride, tetraethylene glycol bis tosylate (step 4).
   The product of step 3 **(3)** is dissolved in dry DMF under nitrogen atmosphere, cooled to 0-5 °C and added with sodium hydride (1.1 eq.) portionwise. After stirring 1 h at room temperature, the mixture is again cooled to 0-5 °C and added with a solution of compound **4** (0.5 eq.) in DMF (3 vol). After stirring for 12 h at room temperature, the mixture is added with water and extracted with ethyl acetate. After removal of the solvent the mixture is purified by chromatography eluting with hexanes/ethyl acetate. Yield 40%.
*Step 6:*
   Reagents: Dichloromethane, trifluoroacetic acid. Not purified.
   The product of step 5 **(5)** is dissolved in dichloromethane (10 vol) and added with trifluoroacetic acid (2 vol). After stirring for 3 h at room temperature, the solvent is removed under vacuum and the residue used for the next step without further purification. Yield quantitative.
*Step 7:*
   Reagents: DMF, 5-methyl-tetrazole, dibenzyloxy-diisopropylphosphoroamide, then H₂O₂.
   The product of step 6 **(6)** is dissolved in dry DMF (10 vol) under nitrogen atmosphere, added with 5-methyltetrazole (9 eq.) and cooled to -10 °C, followed by drop-wise addition of dibenzyloxy-diisopropylphosphoroamide (6 eq.). After stirring for 12 h at room temperature, the mixture is cooled to 0-5 °C and added drop-wise with H2O2 30% (9 eq.). After stirring 1 h at room temperature, water is added and the mixture is extracted with ethyl acetate. After removal of the solvent, the residue is purified by column chromatography eluting with chloroform/methanol 96/4. Yield 40%.
*Step 8:*
   Reagents: H2, Pd/C. Magnesium salt formation with Magnesium ethoxide.
   The product of step 7 **(7)** is dissolved in methanol (15 vol), added with Pd/C 10% and hydrogenated at room temperature and room pressure for 24 hs. After filtration of the catalyst, the solvent is removed under reduced pressure and compound 8 as acid is obtained. Yield quantitative. The formation of the magnesium salt is performed by adding an aqueous solution of magnesium ethoxide to the aqueous solution of compound 8, followed by distillation of the solvent under vacuum.

### Example 2: Synthesis of (OEG₄)₄-(IP5)₄

Compound 5 (PMB = p-methoxybenzyl; Fig. 7) was prepared in three steps starting from myoinositol-1,3,5-orthoformate, as described in the synthesis of OEG₄-(IP5)₂ (Example 1; Fig. 4).

*Reagents of step A (**Fig. 7**):* P-Toluenesulfonyl chloride; dichloromethane, triethyl amine. Column chromatography. Yield 80%.

*Reagents of step B (**Fig. 7**):* Trimethylol propane, DMF, sodium hydride. Column chromatography. Yield 45%.

*Reagents of step C (**Fig. 7**):* Methanol, H₂, Pd/C. Yield 80%.

*Reagents of step D (**Fig. 7**):* P-Toluenesulfonyl chloride; dichloromethane, triethyl amine. Column chromatography. Yield 70%.

*Reagents of step E (**Fig. 7**):* Compound 5 as shown in Fig. 7, DMF, sodium hydride. Column chromatography. Yield 20%.

*Reagents of step F (**Fig. 7**):* Dichloromethane, trifluoroacetic acid. Yield quantitative.

*Reagents of step G (**Fig. 7**):* DMF, dibenzyl-N,N-diisopropylphosphoramidite, 5-methyltetrazole. Column chromatography. Yield 30%.

*Reagents of step H (**Fig. 7**): Methanol, H2, Pd*/*C. Yield 90%. Then sodium salt formation, quantitative.*

### Example 3: Inhibition of CaOx crystallization

For comparison of the compounds' efficacy to inhibit calcium oxalate (CaOx) crystallization a simple in vitro assay in urine was used. In brief, human urine (pH 6.2) was mixed with compound, followed by the addition of 1mM sodium oxalate inducing CaOx crystallization. After 7 hours of incubation at room temperature crystallization was measured by light microscopy, employing an automated image analysis to detect, classify and quantify the CaOx crystal types formed, namely CaOx monohydrate (COM) and CaOx dihydrate (COD).

The inventors found that all three novel divalent IP5 structures showed similar CaOx inhibitory efficacy in vitro with complete inhibition of CaOx crystallization at 4 µmol/L (Fig.1). The length of the linker did not have an important impact on inhibitory efficacy. Compared to the previously developed, most potent compound IP5-mono-PEG2 (OEG₂-IP5) (PCT/EP2012/004088 & PCT/EP2016/080657), OEG₄-(IP5)₂ showed a 2500-fold increase in potency to inhibit CaOx crystallization to below 50% of the control (Fig. 1).

The inventors observed that first, the compounds at sub-inhibitory concentrations lead to a shift in crystallization from COM towards COD, further increasing concentrations of compound can then completely inhibit CaOx growth with an unexpected efficiency. All tested divalent IP5 structures showed complete CaOx inhibition in the in vitro assay at low micromolar concentrations, which are expected to be achieved in vivo (Fig.2, Table 1).

The inventors showed that the efficacy to inhibit CaOx crystallization is dependent on the number of phosphate groups on the molecule and that complete inhibition of crystallization was observed with all OEGₓ-(IP5)₂ compounds at 4 µmol/L (Table 1). Furthermore, IP6 and other compounds tested up to 30 µM or 100 µM, respectively, did not lead to complete CaOx inhibition (Fig.3, Table 1). Minimal impact of the length of the OEG chain or length of linker on total CaOx crystallization, was observed mostly affecting COM-COD formation dynamics. Moreover, decreasing the pH to 5.5 reduces the activity of OEG₂-IP5 more drastically than of OEG₄-(IP5)₂. The compound OEG₂-IP5 at 100 µM did not result in < 50 % complete inhibition or COM inhibition. In addition, the compound OEG₄-(IP5)₂ retained COM inhibition at 0.3 µM and 50% complete inhibition at 2.4 µM (Fig.1, Table 1). The compound (OEG₄)₃-(IP5)₃ displayed COM inhibition at 0.04 µM and complete inhibition at 2.4 µM (Fig. 8, Table 1).

**Table 1**

| | Compound | Complete inhibition (i.e < 5% total area) (µM) | 50% complete inhibition (< 50% total area) (µM) | COM inhibition (< 5% of total area) (µM) |
|---|---|---|---|---|
| pH 6.2 | IP6 | >30 | 30 | 3 |
| | OEG₂-IP5 | >100 | 100 | 11 |
| | OEG₁₁-IP5 | >100 | 11 | 100 |
| | (OEG₂)₂-IP4 | >100 | >100 | >100 |
| | OEG₂-(IP5)₂ | 4 | 0.3 | 0.3 |
| | OEG₄-(IP5)₂ | 4 | 0.04 | 0.3 |
| | OEG₈-(IP5)₂ | 4 | 2.4 | 2.4* |
| | (OEG₄)₃-(IP5)₃ | 2.4 | 2.4 | 0.04 |
| | Citrate | >100 | >100 | >100 |
| pH 5.5 | OEG₂-IP5 | >100 | >100 | >100 |
| | OEG₄-(IP5)₂ | >4 | 2.4 | 0.3 |
| | * High COD crystallization - possible misclassification as COM at 300nM | | | |

### Example 4: OEG₄-(IP5)₂ inhibits CaOx crystallization by early-stage precursor stabilization

Scanning electron microscopy (SEM) experiments of early stage crystallization at t = 10 min, revealed the presence of both, COM shaped- and round nanosized particles, while with 500 nM OEG₄-(IP5)₂ only nanosized particles were detected (Fig. 9B). Nanosized particles were still present at t = 1 h with 0.5 - 10 µM of inhibitor, in contrast to micrometer sized COM crystals being predominant in the control sample, suggesting the stabilization of early stage, nanosized CaOx particles by OEG₄-(IP5)₂.

SEM experiments further confirmed a dose-dependent shift from predominately COM towards COD crystallization, and a COD face-specific growth inhibition with OEG₄-(IP5)₂ (Fig. 9A). Taken together these results indicate that OEG₄-(IP5)₂ alters the crystallization process at several stages, namely crystal nucleation and growth kinetics, as well as crystal polymorphism and shape.

### Example 5: OEG₄-(IP5)₂ blocks CaOx adhesion to renal epithelial cells in vitro more efficiently than OEG₂-IP5

Anti-adhesive effects of chosen IP6 analogues on CaOx crystals to renal proximal tubular epithelial cells (RPTECs) *in vitro* were investigated by differential interference contrast microscopy. Comparison of OEG₄-(IP5)₂, OEG₂-IP5 and (OEG₂)₂-IP4 showed that concentrations of 200 nM, 4 µM and above 100 µM, respectively, lead to < 25 % of CaOx adhesion compared to the control (Fig. 10A-D). Citrate treatment did not lead to any detectable protection against CaOx adhesion up to 100 µM. Additionally, OEG₄-(IP5)₂ not only inhibited CaOx adhesion, but also reversed the binding of prebound CaOx crystals at low micromolar concentrations (Fig. 10E).

### Example 6: Cyto-protective effects of OEG₄-(IP5)₂ on CaOx induced cellular injury

RNA sequencing of RPTECs showed a similar gene expression profile between medium control, OEG₄-(IP5)₂ control and cells treated with CaOx premixed with OEG₄-(IP5)₂, while CaOx treatment alone induced drastic changes (Fig. 11A). Gene set enrichment analysis of differently expressed genes in the CaOx over medium samples revealed alteration of genes mainly involved in immune and inflammatory responses, which coincides with literature, as well as structural changes (e.g. microtubule organization) and protein modification and signaling effects (e.g. peptidyl-glutamic acid modification, ER-nucleus signaling pathway) (Fig. 11B). Comparing the top 10 enriched gene sets of differentially expressed genes in CaOx over medium samples versus CaOx over CaOx - OEG₄-(IP5)₂ samples showed similar changes, indicating that premixing OEG₄-(IP5)₂ with CaOx can prevent CaOx induced transcriptomic changes (Fig. 11B). Elevated expression of TNF alpha and TGF beta signaling pathway genes, cell surface glycoprotein, C-X-C motif chemokine ligand and complement cascade genes, in the CaOx group compared to all other treatment groups were detected (Fig. 11C). Further, CaOx crystal treatment triggered plasma membrane damage, as evidenced by ethidium homodimer-1 staining, which was prevented by OEG₄-(IP5)₂ in a dose-dependent manner (Fig.11 D,E).

### Materials and methods

### Materials

Compounds were custom synthesized by Chimete Srl (Tortona, Italy). Phytic acid dodecasodium salt was purchased from Biosynth AG (Thal, Switzerland). Pooled human urine was purchased from Lee Biosolutions (Maryland Heights, Missouri). Oxalate assay kit (MAK315), calcium colorimetric assay kit (MAK022), Bis-Tris, sodium oxalate, formalin solution neutral buffered 10% and Mowiol 4-88 were purchased from Sigma-Aldrich (St.Louis, Missouri). Sodium chloride and calcium chloride dehydrate were obtainted from Merck (Kenilworth, New Jersey). Calcium oxalate monohydrate was purchased from abcr (Karlsruhe, Germany). 8-well glass bottom slides (80827) were purchased from ibidi (Martinsried, Germany). Trisodium citrate dihydrate analytical grade, Nunc Lab-Tek II chamber slides, cell culture plates, Live/Dead Viability/Cytotoxicity kit for mammalian cells and phosphate buffered saline (PBS) were purchased from from Thermo Fisher Scientific (Rochester, NY). RPTEC/TERT1 cells, ProxUp basal medium and supplements were obtained from Evercyte (Vienna, Austria). RNeasy kit was purchased from Quiagen (Hilden, Germany) and TrueSeq RNA kit from Illumina (San Diego, California).

### CaOx screening assay and analysis

Human urine was stored at -20°C in 50 mL aliquots. After thawing, aliquots were centrifuged, filtered using a 0.45-µm syringe filter and pH set to 6.2. Oxalate concentrations of human urine samples were determined using the Oxalate Assay kit following the kit protocol, and found to be < 100 µM for the samples used. Dilutions of sodium and compounds were prepared in Bis Tris buffer (50 mM, 150 mM NaCl, pH 6.2). Urine was mixed with compound in Eppendorf tubes, and subsequently sodium oxalate was added. The final assay mixture of 1 mL total volume contained 90% urine, 5% sodium oxalate (1 mM) and 5% compound dilution. Immediately upon sodium oxalate addition, the final assay mixture was vortexed and added to the imaging slide (8 well glass bottom chamber, ibidi). 400 µL per well were added and two wells for each sample were prepared. Crystallization was assessed after 7 h incubation at room temperature, using a Leica CTR6000 microscope (Leica Microsystems, Wetzlar, Germany) in brightfield mode. For visualization, images with a 40x objective were used. For quantification 2 wells /sample with five 20x objective images each were used. Without addition of sodium oxalate no crystallization was observed.

Inhibition of seeded crystals was evaluated in a similar manner. Crystallization was induced with 1 mM sodium oxalate in urine as above followed by compound addition after 1.5 h incubation at room temperature directly to each well. Samples were imaged before compound addition at t = 1.5 h and afterwards at t = 7 h total incubation time.

Quantification and classification of crystals was performed in Matlab (version R2016b or R2018b). In brief, crystals were segmented using edge detection and watershed algorithms and subsequently shape, intensity and texture features for each crystal were extracted. The extracted features served as input for a semi-supervised classification approach to distinguish COM (class 0), COD (class 1), not determined structures (class 2) and background noise (class 3). A cubic support vector machine (SVM) classifier was trained and subsequently used for classification of testing images.

All testing images were processed using the Batch Processing App. Efficacy of compounds to inhibit CaOx crystallization was compared by analysing the total area per field of view for each crystal type. The sum of the total area of the three crystal classes - COM, COD and unclassified, was normalized to the control within each experiment (N = 3). Due to the inherent variability of the crystallization process itself, normalization within each experiment decreased the effect of inter-experimental variability.

### Scanning electron microscopy

CaOx crystals for SEM were prepared in Bis Tris buffer (50 mM, 150 mM NaCl, pH 6.2) containing 1 mM NaOx, 2 mM CaCl₂ and the indicated concentration of compound. 12 mm round glass coverslips were placed at the bottom of 24 well plates. First, stock solutions of 20x final concentrations of CaCl₂ and inhibitor, and 10x final concentration of NaOx were prepared in the Bis Tris buffer. Assay mixture was prepared in Eppendorf tubes by first adding 800 µL of Bis Tris buffer, followed by addition of 50 µL CaCl₂ (20x concentration) and 50 µL of inhibitor (20x concentration) and vortexing. Then 100 µL NaOx (10x final concentration) was added, the assay mixture was vortexed and immediately added to the prepared 24 well plate. 400 µL per well and 2 wells per sample were prepared. Samples were incubated at room temperature for the indicated time. Example images were taken using a Leica CTR6000 microscope (Leica Microsystems) in brightfield mode before samples were washed once with double distilled water (ddH₂O) and drying at room temperature. Samples were imaged by brightfield microscopy again after drying to confirm little effects of the drying process on crystal morphology. After drying, glass coverslips were mounted on SEM stubs with silver paint and coated with a 6 nm layer of Platinum/Palladium using a CCU-010 Metal Sputter Coater (Safematic, Bad Ragaz, Switzerland). Samples were imaged using a Magellan 400 FEI SEM microscope (ThermoFisher Scientific, Rochester, NY) in the secondary electron mode using the TLD detector.

### Cell experiments

RPTEC/TERT1 human proximal tubule cells (Evercyte) were cultured in ProxUp basal medium (Evercyte, DMEM/Ham's F12, Hepes buffer and GlutaMAXTM) mixed with ProxUp supplements (Evercyte) at 37°C at 5% CO₂ according to the manufacturer's recommendations. Cells were regularly tested for Mycoplasma infection.

Cell viability was assessed using the Live/Dead Viability/Cytotoxicity kit (ThermoFisher Scientific). Cells were cultured in 24 well plates and 48 h after seeding (upon reaching confluence) treated with 150 µg/cm² Calcium oxalate monohydrate (abcr) in ProxUp basal premixed with compound. After 48 h treatment, cell viability was determined by staining with the Live/Dead cytotoxicity kit (ThermoFisher Scientific), according to the kit protocol. Images were obtained by epifluorescence microscopy with a Leica CTR6000 microscope (Leica Microsystems, 3 wells/sample, 3 images/well) and the number of red fluorescence dots was quantified.

For quantification of CaOx adhesion to RPTEC cells, cells were cultured on 8-well Nunc Lab Tek chamber slides (ThermoFisher Scientific), and upon confluence at 48 h treated with COM (abcr) premixed with compound in ProxUp basal medium (pH set to 6.9) for 30 min at 37°C, 5% CO₂. Cells were washed twice with phosphate buffered saline (PBS, pH 7.4), fixed with 10 % neutral buffered formalin for 15 min, and rinsed with PBS, followed by a final rinse with ddH₂O. The chamber of the slide was removed and cells mounted with Mowiol. Images were obtained with a Leica CTR6000 microscope (Leica Microsystems) in DIC mode, 63x oil objective (3 wells per sample; 8 images per well) and crystal occupied area quantified.

### RNA sequencing

Cells were cultured as described in the cell viability assay. Four treatment groups consisted of a medium control (ProxUp basal), 150 µg/cm² Calcium oxalate monohydrate (abcr) only, 150 µg/cm² Calcium oxalate monohydrate (abcr) premixed with 10 µM OEG₄-(IP5)₂ and 10 µM OEG₄-(IP5)₂ only. Total RNA was extracted using the RNeasy kit (Quiagen) according to the manufacturer's protocol. mRNA was purified and RNAseq library was preprared using the TrueSeq RNA kit. Sequencing was performed on a Novaseq 6000 (Illumina). Reads were aligned to the human reference genome GRCh38.p10 using the STAR tool and transcripts quantified using the Kallisto program. Ensembl release 91 was used for the gene model definitions. For the heatmap and hierarchial clustering of significantly different genes the log2fold changes in comparison to the mean of all samples was calculated and log2fold changes > 4 were set to 4. Gene set enrichment analysis was performed on Webgestalt.org (v2019) ⁴¹. Input for GSEA were gene lists with the ensemble gene id and a ranking score (-log₁₀ * p-value *sign (log₂ratio)) as a metric for differential expression between two treatment groups. Differential expressed genes were compared to the gene ontology - biological process functional database and as a reference set the human genome - protein coding was used. For comparison of expression levels of selected genes the normalized gene count was used.

### Data analysis

Statistical analysis and graphs were prepared using GraphPad Prism (La Jolla, California), unless otherwise stated. Data are expressed as mean + SD. Ordinary one-way ANOVA testing followed by post-hoc Dunnett's multiple comparison was used for comparison of treatment to control group. Image analysis was carried out using Matlab version R2018b and R2016b (MathWorks, Natick, Massachusetts) unless otherwise stated.

## Claims

1. A compound comprising, particularly consisting of, two or more cyclohexanolpentakisester moieties described by a general formula (I) linked by a common central linker L 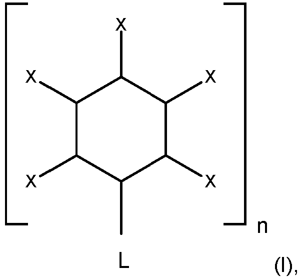
wherein X is OPO₃²⁻, L is a common central linker to which n individual moieties **characterized by** the formula in brackets are attached, and n is an integer selected from 2 and 3,
wherein the common central linker L has a molecular weight <1000 g/mol, and wherein L comprises or essentially consists of a linear or branched poly(ethylene glycol) or polyglycerol.

2. The compound according to claim 1, wherein any one of the cyclohexanolpentakisester moieties described by a general formula (I) is independently selected from a moiety described by general formulae (Ia) or (Ib), 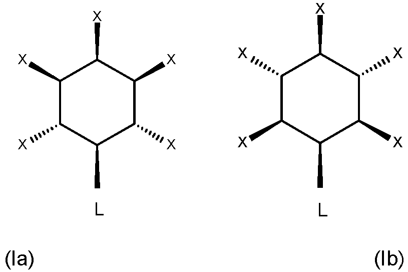 wherein L and X have the meaning defined in claim 1.

3. The compound according to claim 1 or 2, wherein the compound is **characterized by** a general formula (II), (IIi), (IIii), or (IIiii): 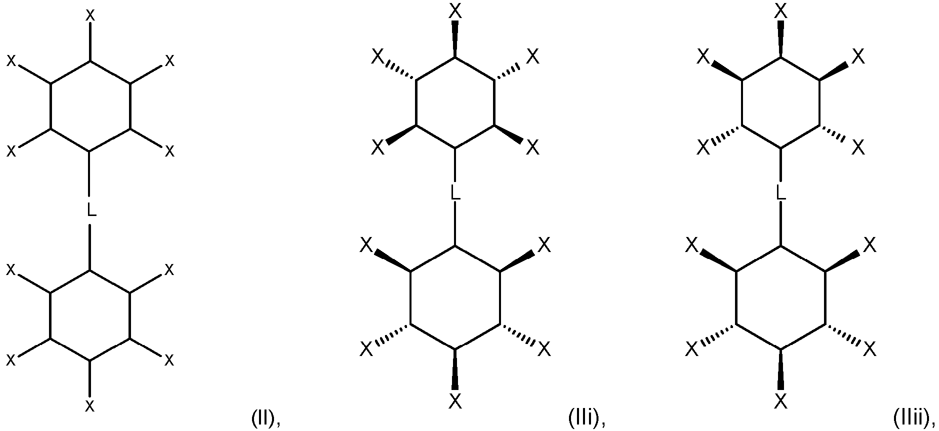 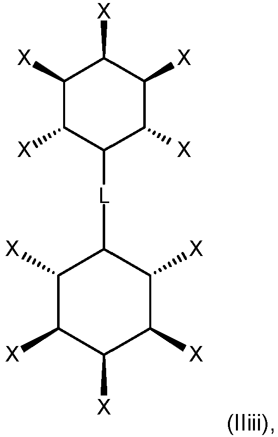
wherein X is OPO₃²⁻, and
wherein L is selected from -(O-CH₂-CH₂)ₘ-O- or -(O-CH₂-CH(OH)-CH₂)ₘ-O-, with m having a value between 1 and 12.

4. The compound according to any one of the preceding claims, wherein the compound is **characterized by** formula (IIa), (IIb), (IIc), IId), (IIe), (IIf), (IIg), (IIh), (IIi), (IIk), (IIm), or (IIn) 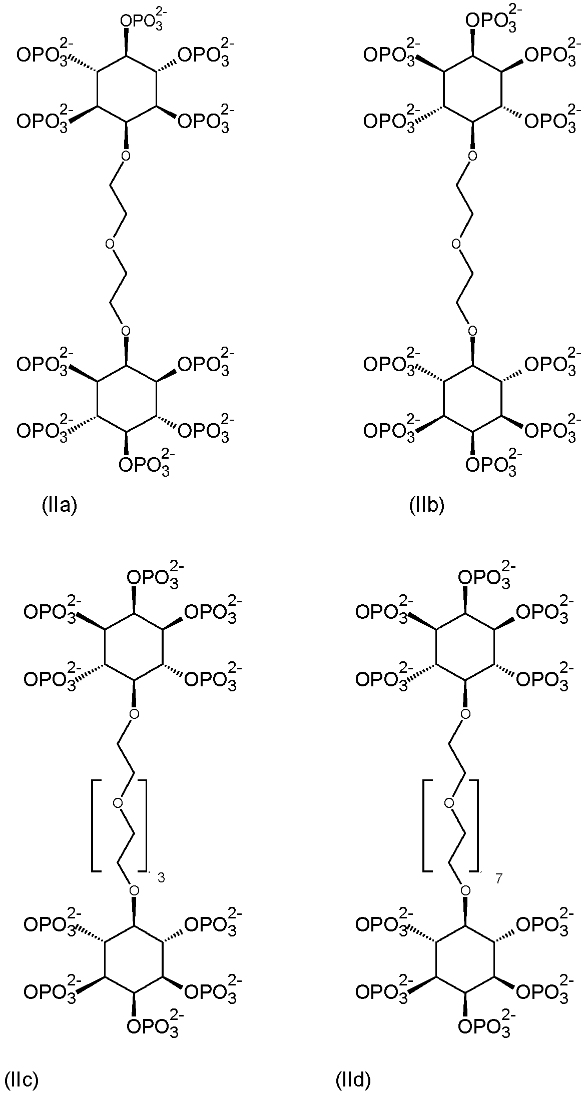 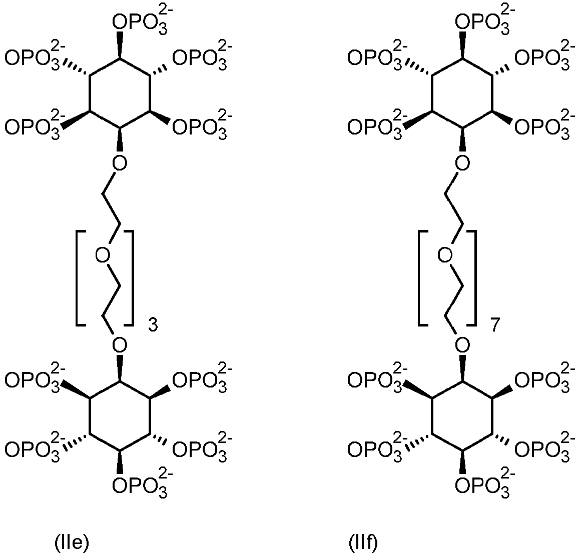 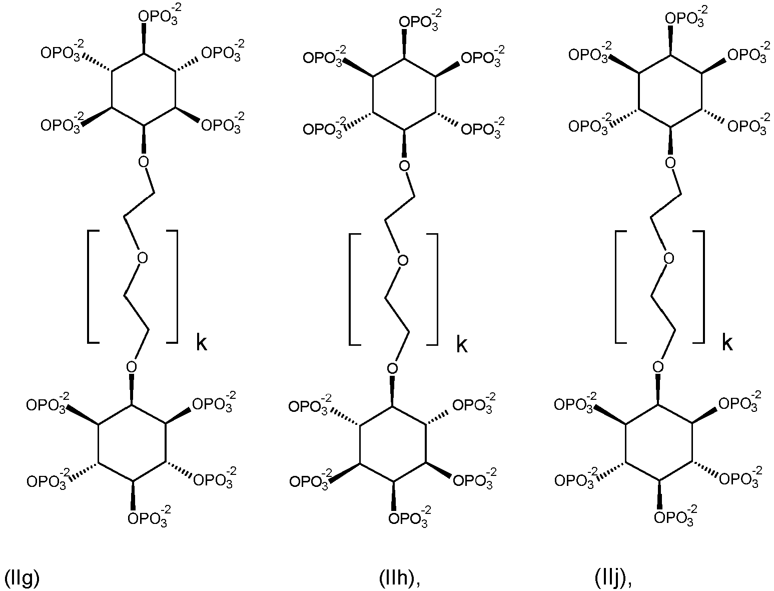 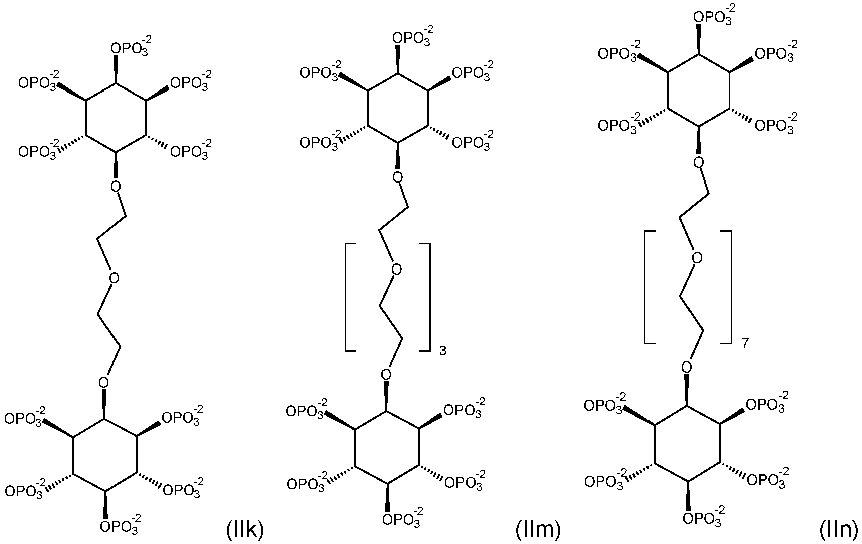 wherein k is an integer selected from 1, 2, 3, 4, 5, 6, 7, 8, 9, 10, 11 and 12.

5. The compound according to claim 1 or 2, wherein the compound is **characterized by** a general formula (III) 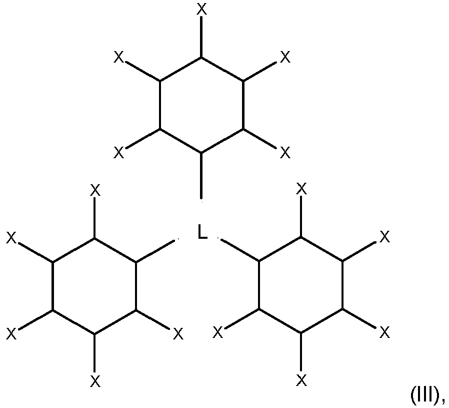
wherein each X has the meaning indicated above and
wherein the linking moiety L is 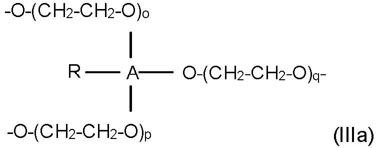 or 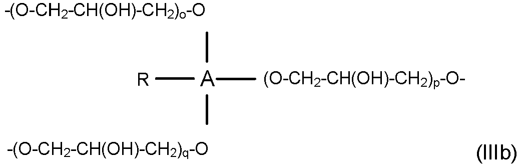 or 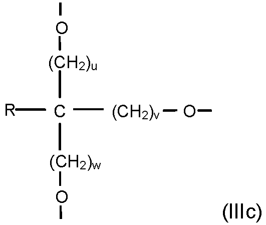 and wherein
- o, p and q independently of each other have a value between 1 and 12 and the sum of o, p and q is ≤ 30,
and
- A is selected from a carbon atom (C), a hydroxy-, amino-, halogen- or carboxy-substituted or an unsubstituted C₄ to C₇ cycloalkyl moiety or a hydroxy-, amino-, halogen- or carboxy-substituted or an unsubstituted aryl and
- R is selected from H and C₁-C₃ unsubstituted or N-, O and/or halogen substituted alkyl,
or
- u, v and w independently of each other have a value between 1 and 12, and the sum of u, v and w is ≤ 30, and R is selected from H and C₁-C₃ unsubstituted or N-, O and/or halogen substituted alkyl.

6. The compound according to claim 5, wherein R is H or C₁-C₃ unsubstituted alkyl.

7. The compound according to claim 5 or 6, wherein o, p and q, or u, v and w, independently of each other have a value between 2 and 4 and the sum u, v and w is ≤ 12, and the sum o, p and q is ≤ 12.

8. The compound according to claim 5,
wherein the compound is **characterized by** formula (Illd) or (Ille):

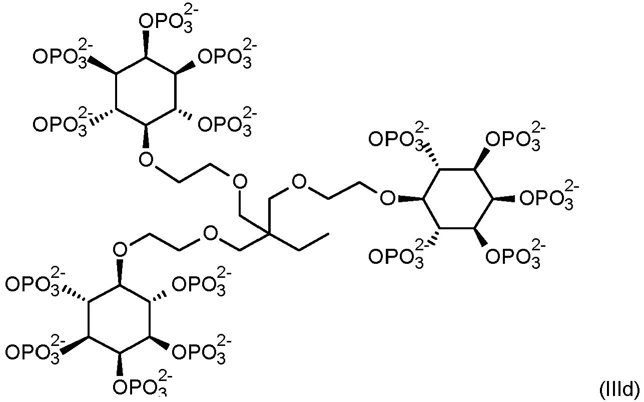

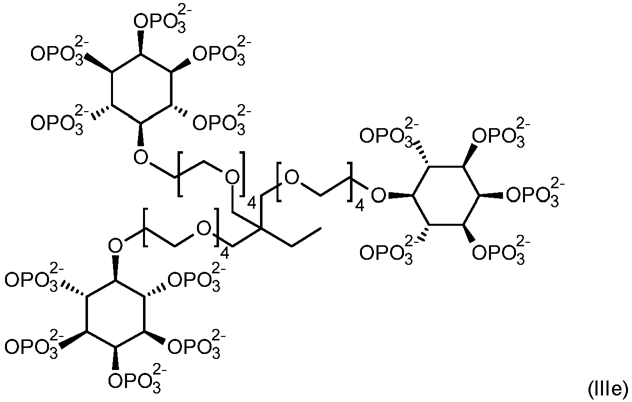

9. A compound according to any one of the preceding claims for use as a medicament.

10. A compound according to any one of the preceding claims
for use in treatment or prevention of a condition related to pathological crystallization selected from nephrocalcinosis or nephrolithiasis due to pathological calcium oxalate or phosphate crystallization in a patient, primary and secondary hyperoxaluria, Dent disease, Bartter's syndrome, distal renal tubule acidosis, neurogenic bladder, autosomal dominant polycystic kidney, calcium oxalate kidney stone formation, struvite stones and renal calcification.

11. The compound for use according to claims 10, wherein the condition related to pathological crystallization is associated with formation of oxalate, phosphate and/ or calcium precipitates.

12. The compound for use according to any of claims 10 or 11, wherein the compound is formulated for intravenous, intraperitoneal, intramuscular, intra-arterial, topical, intravesical or subcutaneous administration.

13. A compound described by general formula Va, Vb, Vc, Vla, Vlb or Vlc, 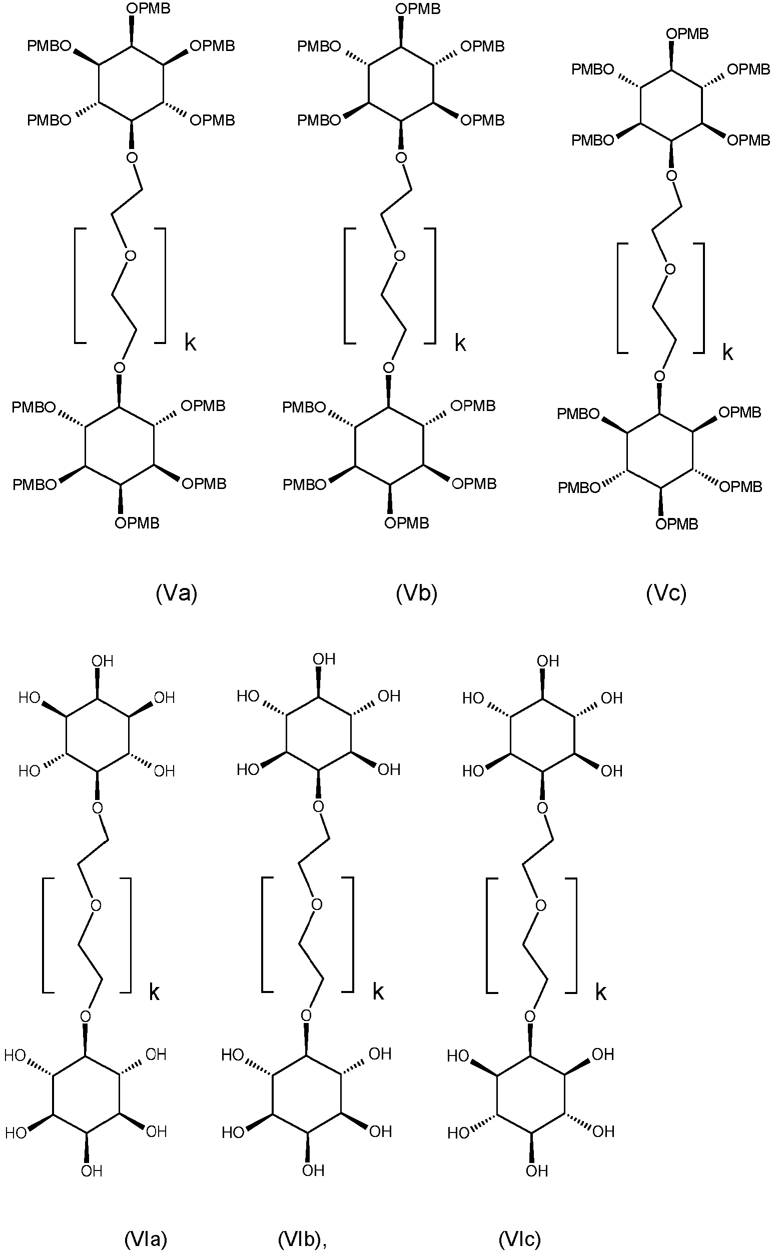 wherein OPMB signifies an O-(bis-paramethoxybenzyl)phosphate moiety and k is an integer selected from 1 to 12.

14. The compound according to claim 13, wherein k is selected from 2, 3, 4, 5, 6 or 7.

## Patentansprüche

1. Verbindung, umfassend, insbesondere bestehend aus, zwei oder mehr Cyclohexanolpentakisester-Einheiten, beschrieben durch eine allgemeine Formel (I), die durch einen gemeinsamen zentralen Linker L verbunden sind, 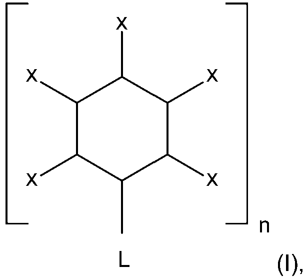
wobei X OPO₃²⁻ ist, L ein gemeinsamer zentraler Linker ist, an den n einzelne Einheiten, **gekennzeichnet durch** die Formel in Klammern, gebunden sind, und n eine ganze Zahl, ausgewählt aus 2 und 3, ist,
wobei der gemeinsame zentrale Linker L ein Molekulargewicht < 1000 g/mol aufweist, und wobei L ein lineares oder verzweigtes Poly(ethylenglykol) oder Polyglycerol umfasst oder im Wesentlichen daraus besteht.

2. Verbindung nach Anspruch 1, wobei jede der durch eine allgemeine Formel (I) beschriebenen Cyclohexanolpentakisester-Einheiten unabhängig aus einer durch die allgemeinen Formeln (la) oder (Ib) beschriebenen Einheit ausgewählt ist 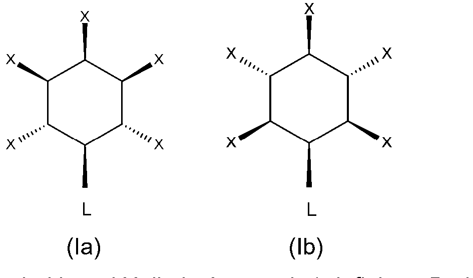 wobei L und X die in Anspruch 1 definierte Bedeutung haben.

3. Verbindung nach Anspruch 1 oder 2, wobei die Verbindung durch eine allgemeine Formel (II), (Ili), (Ilii) oder (Iliii) gekennzeichnet ist: 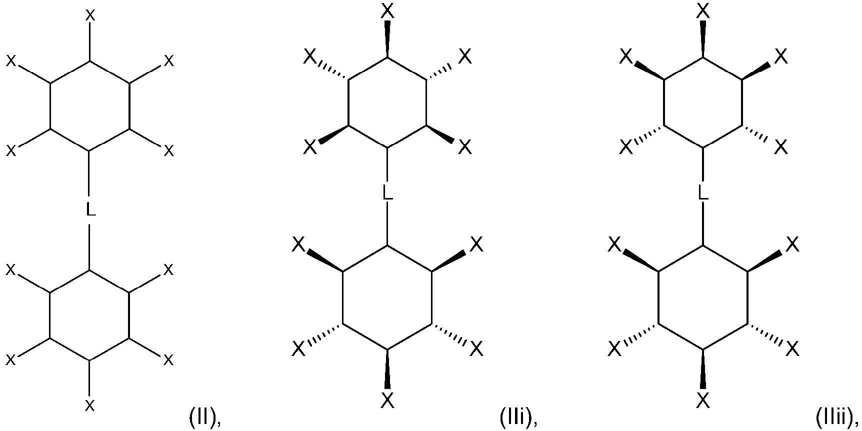 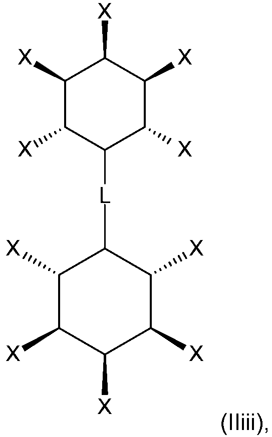
wobei X OPO₃²⁻ ist und
wobei L aus -(O-CH₂-CH₂)ₘ-O- oder -(O-CH₂-CH(OH)-CH₂)ₘ-O- ausgewählt ist, wobei m einen Wert zwischen 1 und 12 aufweist.

4. Verbindung nach einem der vorangehenden Ansprüche, wobei die Verbindung durch die Formel (IIa), (Ilb), (IIc), (IId), (IIe), (IIf), (Ilg), (IIh), (Ili), (IIk), (Ilm) oder (Iln) gekennzeichnet ist: 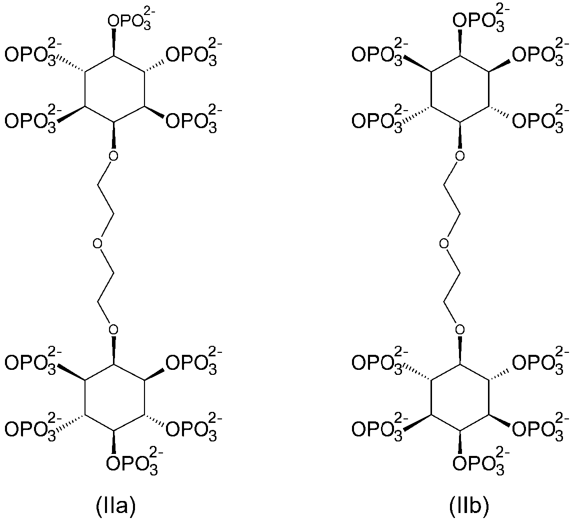 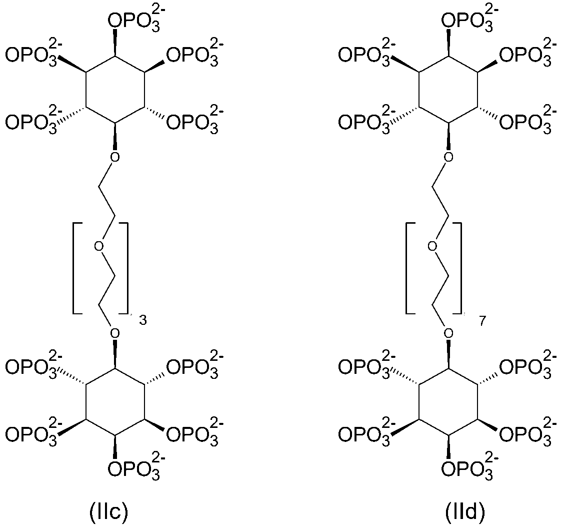 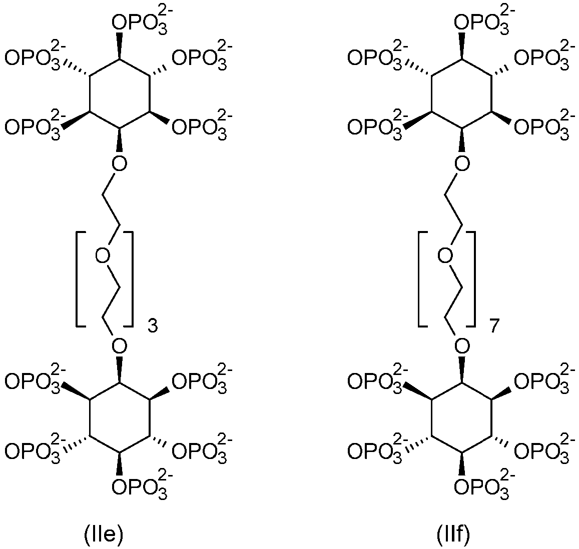 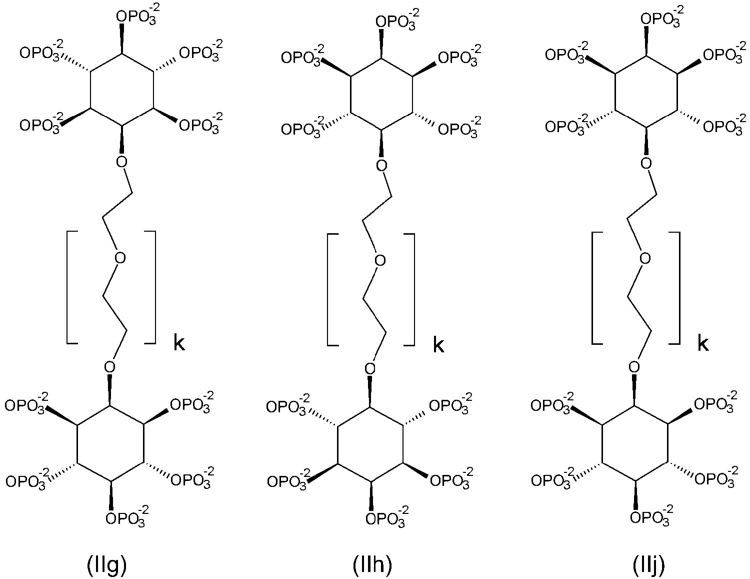 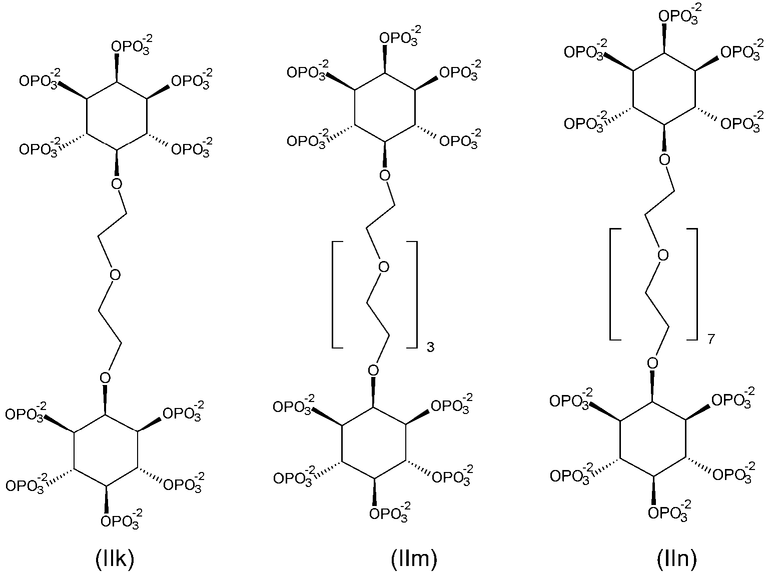 wobei k eine ganze Zahl, ausgewählt aus 1, 2, 3, 4, 5, 6, 7, 8, 9, 10, 11 und 12, ist.

5. Verbindung nach Anspruch 1 oder 2, wobei die Verbindung durch eine allgemeine Formel (III) gekennzeichnet ist 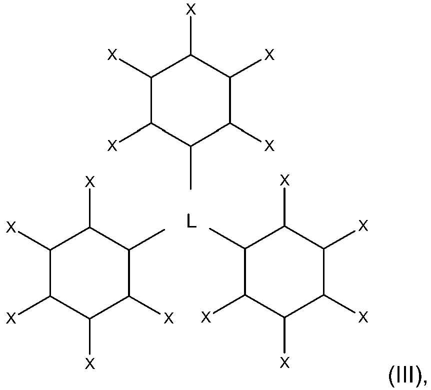
wobei jedes X die vorstehend angegebene Bedeutung hat und
wobei die Verknüpfungseinheit L 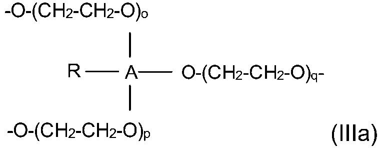 oder 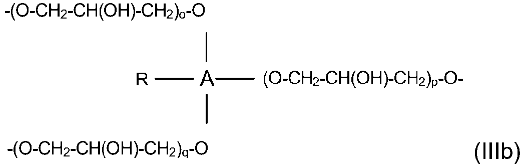 oder 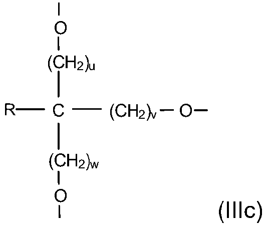 ist,
und wobei
- o, p und q unabhängig voneinander einen Wert zwischen 1 und 12 aufweisen und die Summe von o, p und q ≤ 30 ist,
und
- A ausgewählt ist aus einem Kohlenstoffatom (C), einer hydroxy-, amino-, halogen- oder carboxysubstituierten oder einer unsubstituierten C₄- bis C₇-Cycloalkyl-Einheit oder einem hydroxy-, amino-, halogen- oder carboxysubstituierten oder einem unsubstituierten Aryl und
- R ausgewählt ist aus H und unsubstituiertem oder N-, O- und/oder halogensubstituiertem C₁-C₃-Alkyl,
oder
- u, v und w unabhängig voneinander einen Wert zwischen 1 und 12 aufweisen und die Summe von u, v und w ≤ 30 ist und R ausgewählt ist aus H und unsubstituiertem oder N-, O- und/oder halogensubstituiertem C₁-C₃-Alkyl.

6. Verbindung nach Anspruch 5, wobei R H oder unsubstituiertes C₁-C₃-Alkyl ist.

7. Verbindung nach Anspruch 5 oder 6, wobei o, p und q, oder u, v und w, unabhängig voneinander einen Wert zwischen 2 und 4 aufweisen und die Summe von u, v und w ≤ 12 ist und die Summe von o, p und q ≤ 12 ist.

8. Verbindung nach Anspruch 5,
wobei die Verbindung durch die Formel (IIId) oder (IIIe) gekennzeichnet ist:

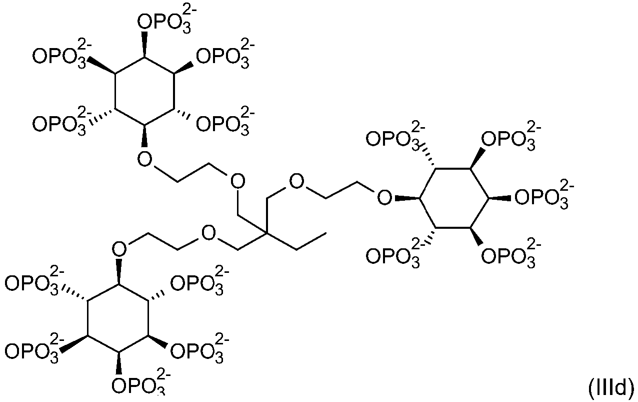

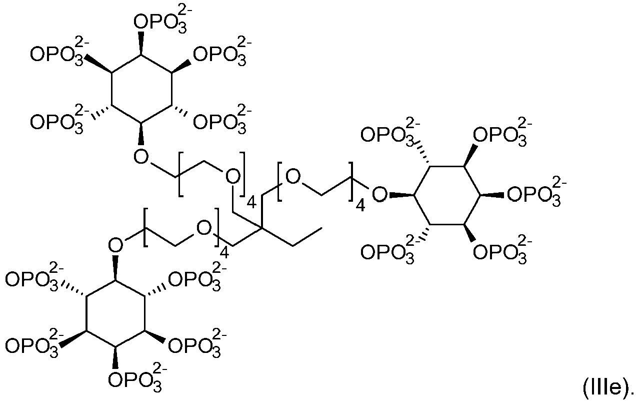

9. Verbindung nach einem der vorangehenden Ansprüche zur Verwendung als Medikament.

10. Verbindung nach einem der vorangehenden Ansprüche
zur Verwendung bei der Behandlung oder Vorbeugung eines mit pathologischer Kristallisation in Zusammenhang stehenden Zustands, ausgewählt aus Nephrokalzinose oder Nephrolithiasis infolge pathologischer Kristallisation von Calciumoxalat oder -phosphat bei einem Patienten, primärer und sekundärer Hyperoxalurie, Dent-Krankheit, Bartter-Syndrom, distaler renaler tubulärer Azidose, neurogener Harnblase, autosomal-dominanter polyzystischer Niere, Bildung von Calciumoxalat-Nierensteinen, Stuvitsteinen und Nierenverkalkung.

11. Verbindung zur Verwendung nach Anspruch 10, wobei der mit pathologischer Kristallisation in Zusammenhang stehende Zustand mit der Bildung von Oxalat-, Phosphat- und/oder Calciumablagerungen verbunden ist.

12. Verbindung zur Verwendung nach einem der Ansprüche 10 oder 11, wobei die Verbindung für die intravenöse, intraperitoneale, intramuskuläre, intraarterielle, topische, intravesikale oder subkutane Verabreichung formuliert ist.

13. Verbindung, beschrieben durch die allgemeine Formel Va, Vb, Vc, VIa, VIb oder VIc, 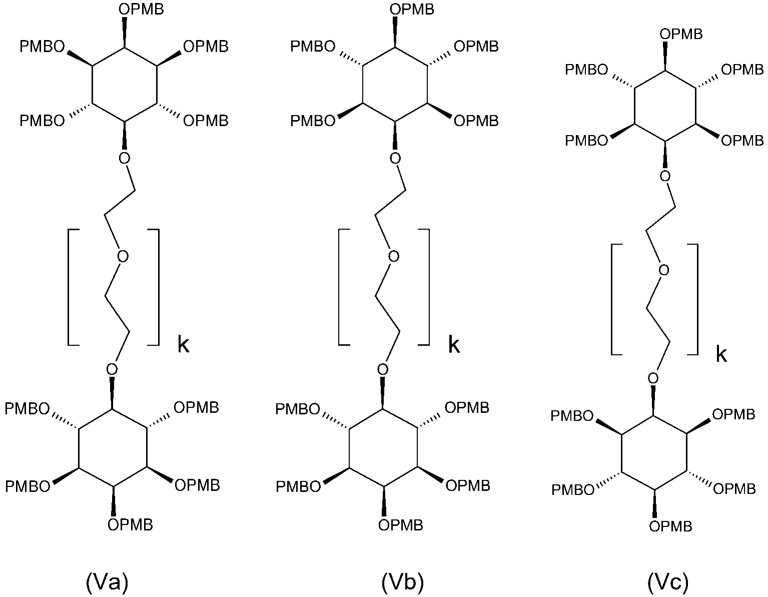 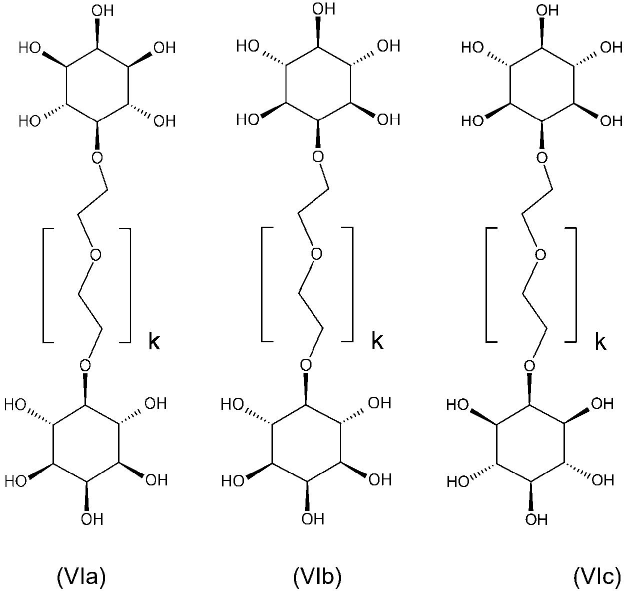 wobei OPMB eine O-(Bis-p-methoxybenzyl)phosphat-Einheit bedeutet und k eine ganze Zahl, ausgewählt aus 1 bis 12, ist.

14. Verbindung nach Anspruch 13, wobei k aus 2, 3, 4, 5, 6 oder 7 ausgewählt ist.

## Revendications

1. Composé comprenant, notamment constitué de, deux groupes caractéristiques d'ester de cyclohexanolpentakis ou plus décrits par une formule générale (I) liés par un liant central commun L 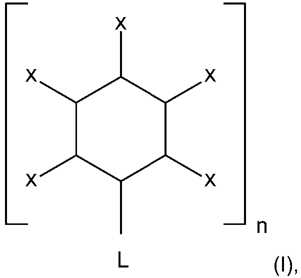
dans laquelle X est OPO₃²⁻, L est un liant central commun auquel n groupes caractéristiques individuels **caractérisés par** la formule entre crochets sont reliés, et n est un entier sélectionné parmi 2 et 3,
dans lequel le liant central commun L a un poids moléculaire <1000 g/mol, et dans lequel L comprend ou est essentiellement constitué de poly(éthylène glycol) ou polyglycérol linéaire ou ramifié.

2. Composé selon la revendication 1, dans lequel l'un quelconque des groupes caractéristiques d'ester de cyclohexanolpentakis décrits par une formule générale (I) est indépendamment sélectionné parmi un groupe caractéristique décrit par les formules générales (Ia) ou (Ib), 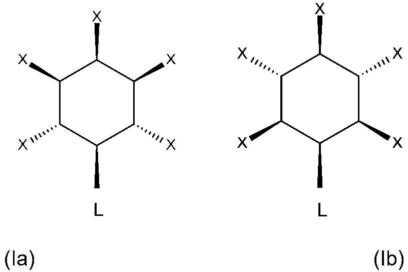 dans lesquelles L et X ont la signification définie à la revendication 1.

3. Composé selon la revendication 1 ou 2, dans lequel le composé est **caractérisé par** une formule générale (II), (Ili), (Ilii) ou (Iliii) : 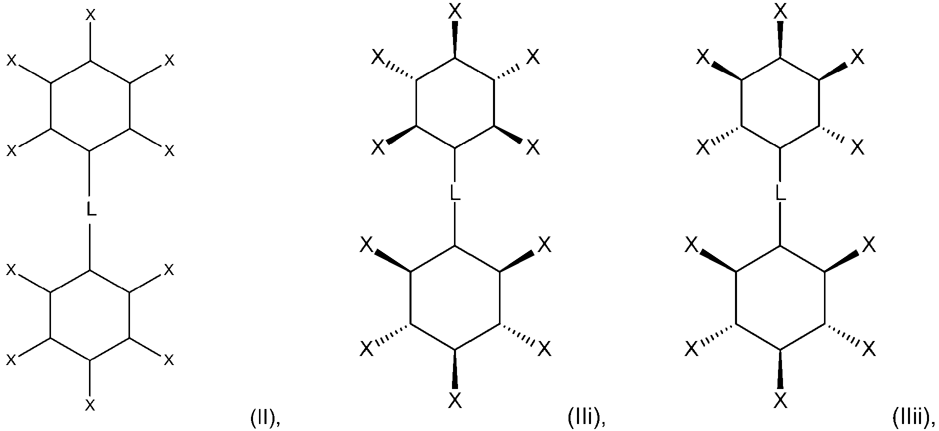 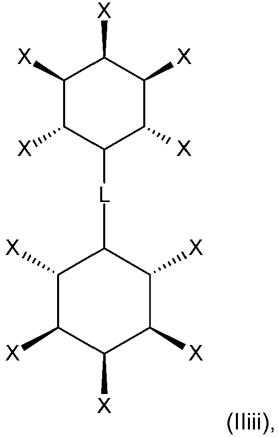
dans lesquelles X est OPO₃²⁻, et
dans lesquelles L est sélectionné parmi -(O-CH₂-CH₂)ₘ-O- ou -(O-CH₂-CH(OH)-CH₂)ₘ-O-, avec m ayant une valeur comprise entre 1 et 12.

4. Composé selon l'une quelconque des revendications précédentes, dans lequel le composé est **caractérisé par** la formule (IIa), (IIb), (IIc), (IId), (IIe), (IIf), (IIg), (IIh), (IIi), (IIk), (IIm) ou (IIn) 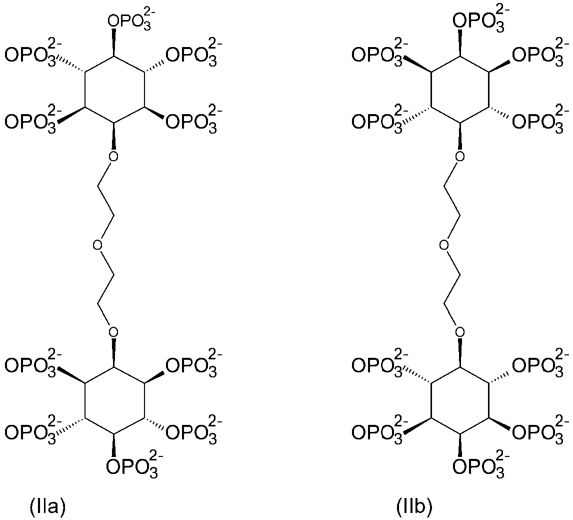 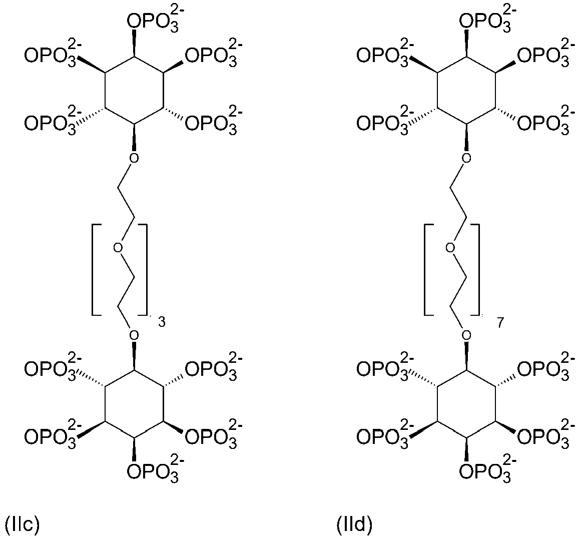 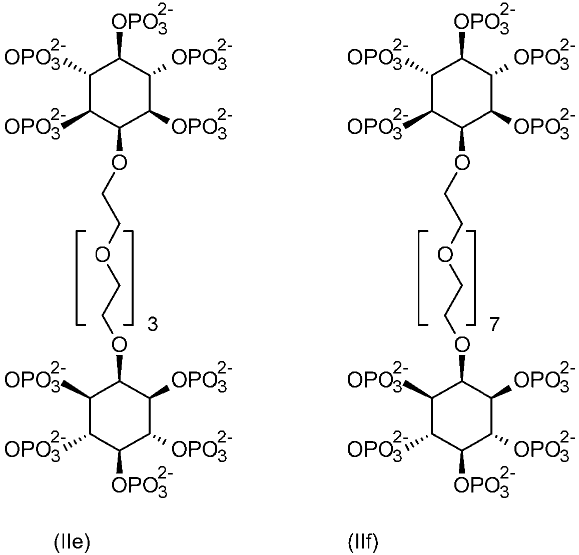 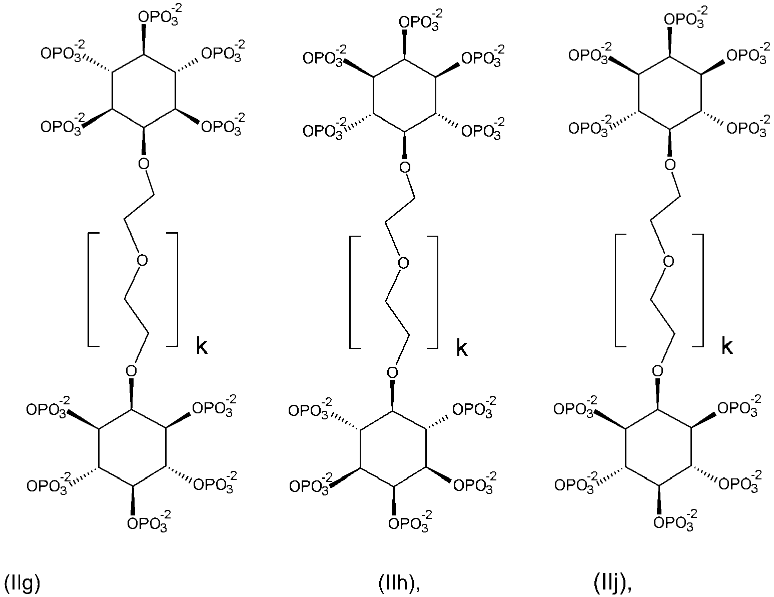 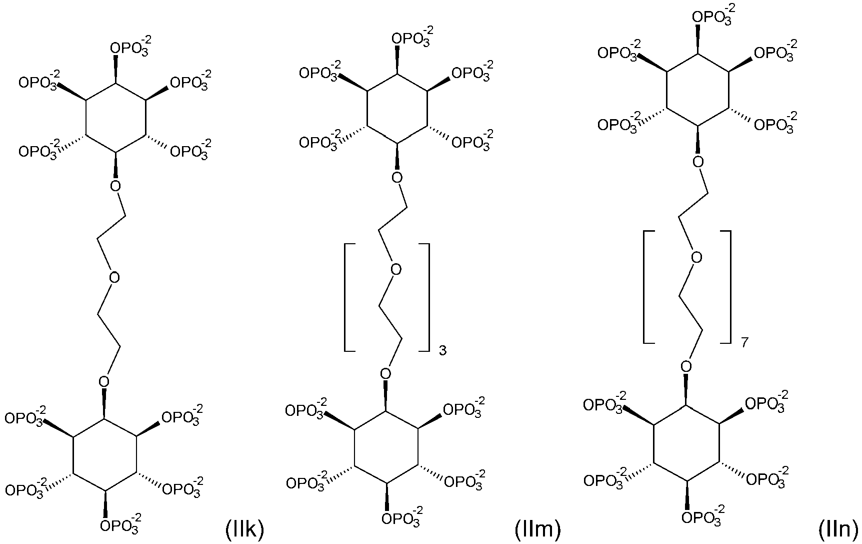 dans lesquelles k est un entier sélectionné parmi 1, 2, 3, 4, 5, 6, 7, 8, 9, 10, 11 et 12.

5. Composé selon la revendication 1 ou 2, dans lequel le composé est **caractérisé par** une formule générale (III) 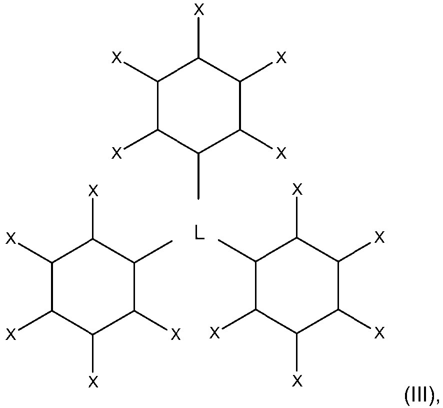
dans laquelle chaque X a la signification indiquée ci-dessus et
dans laquelle le groupe caractéristique de liaison L est 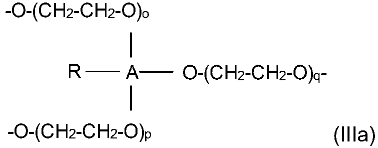 ou 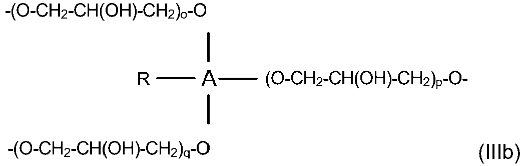 ou 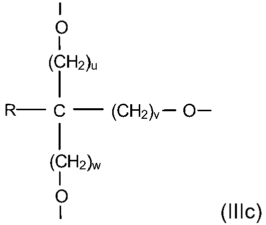 et dans laquelle
- o, p et q indépendamment l'un de l'autre ont une valeur comprise entre 1 et 12, et la somme de o, p et q est ≤ 30,
et
- A est sélectionné parmi un atome de carbone (C), un groupe caractéristique cycloalkyle en C₄ à C₇ substitué par un hydroxy, amino, halogène ou carboxy ou non substitué, ou un aryle substitué par un hydroxy, amino, halogène ou carboxy ou non substitué, et
- R est sélectionné parmi H et alkyle non substitué en C₁-C₃ ou substitué par N, O et/ou un halogène,
ou
- u, v et w indépendamment l'un de l'autre ont une valeur comprise entre 1 et 12, et la somme de u, v et w est ≤ 30, et R est sélectionné parmi H et alkyle non substitué en C₁-C₃ ou substitué par N, O et/ou un halogène.

6. Composé selon la revendication 5, dans lequel R est H ou alkyle non substitué en C₁-C₃.

7. Composé selon la revendication 5 ou 6, dans lequel o, p et q, ou u, v et w, indépendamment l'un de l'autre, ont une valeur comprise entre 2 et 4 et la somme de u, v et w est ≤ 12, et la somme de o, p et q est ≤ 12.

8. Composé selon la revendication 5,
dans lequel le composé est **caractérisé par** la formule (IIId) ou (Ille) :

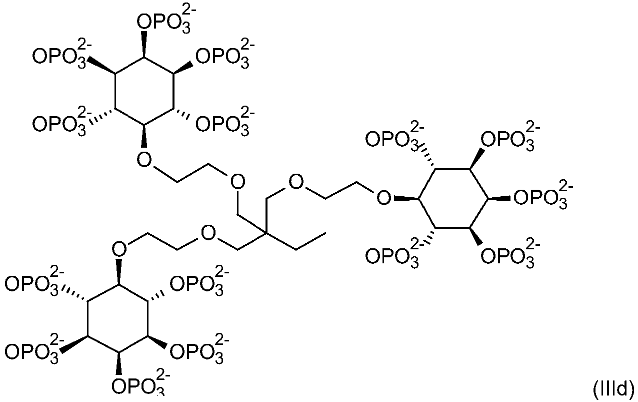

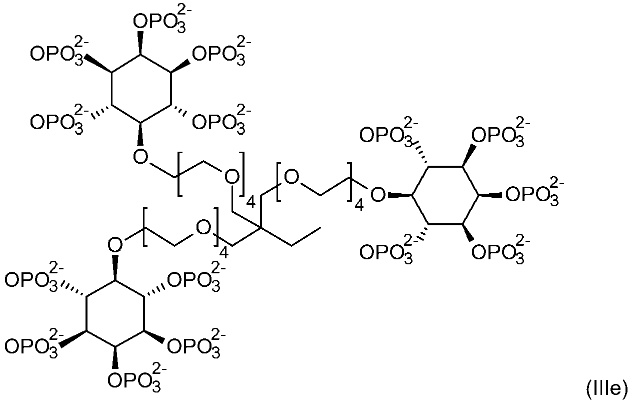

9. Composé selon l'une quelconque des revendications précédentes destiné à être utilisé en tant que médicament.

10. Composé selon l'une quelconque des revendications précédentes destiné à être utilisé dans le traitement ou la prévention d'une affection liée à une cristallisation pathologique sélectionnée parmi la néphrocalcinose ou la néphrolithiase en raison d'une cristallisation d'oxalate ou de phosphate de calcium pathologique chez un patient, d'une hyperoxalurie primaire et secondaire, de la maladie de Dent, du syndrome de Bartter, d'une acidose tubulaire rénale distale, d'une vessie neurogène, d'une polykystose rénale autosomique dominante, d'une formation de calcul rénal d'oxalate de calcium, de calculs de struvite et d'une calcification rénale.

11. Composé destiné à être utilisé selon la revendication 10, dans lequel l'affection liée à une cristallisation pathologique est associée à la formation de précipités d'oxalate, de phosphate et/ou de calcium.

12. Composé destiné à être utilisé selon l'une quelconque des revendications 10 ou 11, dans lequel le composé est formulé pour l'administration par voie intraveineuse, intrapéritonéale, intramusculaire, intra-artérielle, topique, intravésicale ou sous-cutanée.

13. Composé décrit par la formule générale Va, Vb, Vc, Vla, VIb ou VIc, 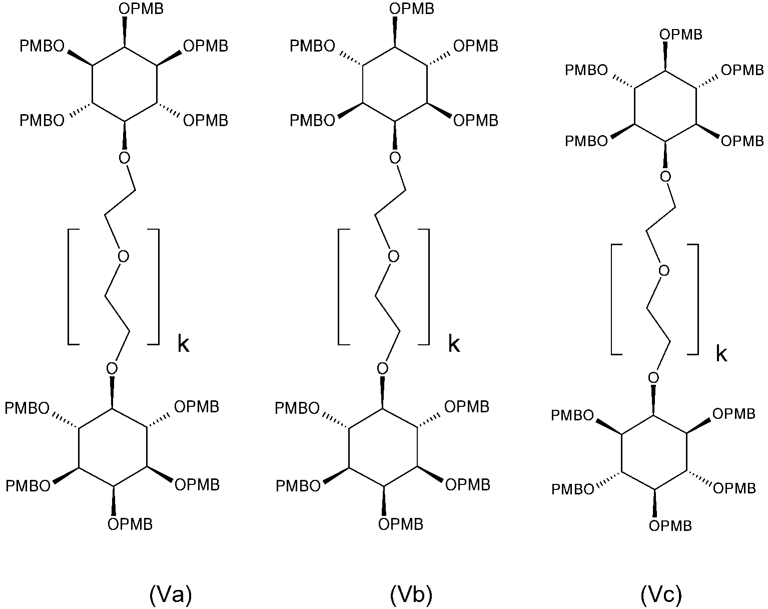 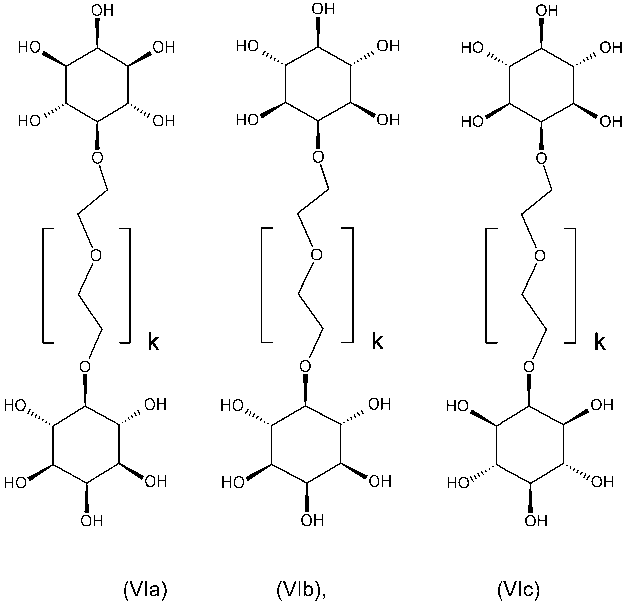 dans lesquelles OPMB signifie un groupe caractéristique O-(bis-paraméthoxybenzyl)phosphate et k est un entier sélectionné parmi 1 à 12.

14. Composé selon la revendication 13, dans lequel k est sélectionné parmi 2, 3, 4, 5, 6 ou 7.
